# FASCICULE DE BREVET EUROPEEN

(11) **EP 2 459 549 B1**
(45) Date de publication et mention de la délivrance du brevet: **19.11.2014**
(21) Numéro de dépôt: 10752090.0
(22) Date de dépôt: 23.07.2010
(51) Int. Cl.: C07D 401/10, C07D 401/12, A61K 31/495

(54) **DÉRIVÉS D'URÉE DE TETRAHYDROQUINOXALINE, LEUR PRÉPARATION ET LEUR APPLICATION EN THÉRAPEUTIQUE**
TETRAHYDROCHINOXALINHARNSTOFFDERIVATE, IHRE HERSTELLUNG UND THERAPEUTISCHE VERWENDUNG
TETRAHYDROQUINOXALINE UREA DERIVATIVES, PREPARATION THEREOF, AND THERAPEUTIC USE THEREOF

(30) Priorité: 27.07.2009 FR 0903687
(43) Date de publication de la demande: 06.06.2012
(73) Titulaire: SANOFI, 75008 Paris (FR)
(72) Inventeur: NAMANE, Claudie, F-75013 Paris (FR); NICOLAI, Eric, F-75013 Paris (FR); PACQUET, François, F-75013 Paris (FR); PASCAL, Cécile, F-75013 Paris (FR); VENIER, Olivier, F-75013 Paris (FR)
(74) Mandataire: Böhm, Brigitte
(86) Numéro de dépôt international: PCT/FR2010/051564
(87) Numéro de publication internationale: WO 2011/012801

(56) Documents cités:
- WO-A2-2008/000950
- WO-A2-2008/000951
- WO-A2-2009/112691
- HUGHES, K.A., WEBSTER, S.P., WALKER, B.R.: "11-Beta-hydroxysteroid dehydrogenase type 1 (11beta-HSD1) inhibitors in Type 2 diabetes and obesity" EXPERT OPIN INVESTIG DRUGS, vol. 17, no. 4, 2008, pages 481-496, XP002566387
- SAIAH EDDINE: "The role of 11beta-hydroxysteroid dehydrogenase in metabolic disease and therapeutic potential of 11beta-hsd1 inhibitors" CURRENT MEDICINAL CHEMISTRY, BENTHAM SCIENCE PUBLISHERS BV, BE, vol. 15, no. 7, 1 janvier 2008 (2008-01-01), pages 642-649, XP002508148 ISSN: 0929-8673

## Description

La présente invention se rapporte à des dérivés d'urée de tétrahydroquinoxaline, à leur préparation et à leur application en thérapeutique.

Les composés selon l'invention modulent l'activité de la 11β-hydroxystéroïde déshydrogénase type 1 (11βHSD1) et sont utiles pour le traitement des pathologies dans lesquelles une telle modulation est bénéfique, comme dans le cas du syndrome métabolique ou du diabète de type 2 non insulino dépendant.

La 11βHSD1 catalyse localement la conversion de glucocorticoïdes inactifs (cortisone chez l'Homme) en glucocorticoïdes actifs (cortisol chez l'Homme) dans différents tissus et organes, principalement le foie et le tissu adipeux, mais aussi dans les muscles, les os, le pancréas, l'endothélium, le tissu oculaire et dans certaines parties du système nerveux central. La 11βHSD1 agit comme un régulateur de l'action des glucocorticoïdes dans les tissus et organes où elle est exprimée (Tomlinson et al., Endocrine Reviews 25(5), 831-866 (2004), Davani et al., J. Biol. Chem. 275, 34841 (2000) ; Moisan et al., Endocrinology, 127, 1450 (1990)).

Les principales pathologies dans lesquelles interviennent les glucocorticoïdes et l'inhibition de la 11βHSD1 sont indiquées ci-après.

### A. Obésité, diabète de type 2 et syndrome métabolique

Le rôle de la 11βHSD1 dans l'obésité, le diabète de type 2 et le syndrome métabolique (aussi connu sous le nom de syndrome X ou syndrome de résistance à l'insuline) où les symptômes incluent l'obésité viscérale, l'intolérance au glucose, la résistance à l'insuline, l'hypertension, le diabète de type 2 et l'hyperlipidémie (Reaven Ann. Rev. Med 44, 121 (1993)) est décrit dans de nombreuses publications. Chez l'Homme, le traitement par la carbenoxolone (un inhibiteur non spécifique de la 11βHSD1) améliore la sensibilité à l'insuline chez des patients volontaires minces et chez des diabétiques de type 2 (Andrews et al., J. Clin. Endocrinol. Metab. 88, 285 (2003)). De plus, les souris dont le gène de la 11βHSD1 a été éteint sont résistantes à l'hyperglycémie induite par le stress et l'obésité, montrent une atténuation de l'induction d'enzymes hépatiques de la néoglucogenèse (PEPCK et G6P) et présentent une augmentation de la sensibilité à l'insuline dans le tissu adipeux (Kotelevstev et al., Proc. Nat Acad. Sci. 94, 14924 (1997) ; Morton et al., J. Biol. Chem. 276, 41293 (2001)). Par ailleurs, les souris transgéniques où le gène de la 11βHSD1 a été surexprimé dans les tissus adipeux présentent un phénotype similaire à celui du syndrome métabolique humain (Masuzaki et al., Science 294, 2166 (2001)). Il est à noter que le phénotype observé existe sans une augmentation du total des glucocorticoïdes circulants, mais est induit par l'augmentation spécifique de glucocorticoïdes actifs dans les dépôts adipeux.

Par ailleurs, de nouvelles classes d'inhibiteurs spécifiques de la 11βHSD1 sont apparues récemment :
- des arylsulfonamidothiazoles ont montré qu'ils amélioraient la sensibilité à l'insuline et réduisaient le niveau de glucose dans le sang de souris présentant une hyperglycémie (Barf et al., J. Med. Chem. 45, 3813 (2002)). De plus, dans une étude récente, il a été montré que ce type de composés réduisait la prise de nourriture ainsi que la prise de poids chez des souris obèses (Wang et Coll. Diabetologia 49, 1333 (2006*)*) ;
- des triazoles ont montré qu'ils amélioraient le syndrome métabolique et ralentissaient la progression de l'athérosclérose chez des souris (Hermanowski-Vosatka et al., J. Exp. Med. 202, 517 (2005)).

### A2. Complications microvasculaires du diabète

La présence des complications chroniques chez le diabétique de type 2 est souvent associée à la sévérité et à la durée du diabète. Les troubles microvasculaires fonctionnels et structuraux expliquent en grande partie le développement de certaines pathologies observées chez le diabétique telles la neuropathie, la rétinopathie, et la néphropathie (Rayman, Diabetes Review 7 :261-274, 1999 ; Gärtner and Eigentler, Clin Nephrol 70 :1-9, 2008 ; Zent and Pozzi, Sem Nephrol 27 :161-171, 2007; Malecki et al., EJCI38 :925-930, 2008). L'élévation chronique de la glycémie, ou l'intolérance au glucose, représentent des facteurs de risque majeurs de ces complications microvasculaires (Robinson Singleton et al. Diabetes 52 :2867-2873, 2003 ; Lachin et al. Diabetes 57: 995-1001, 2008). En permettant un meilleur contrôle de la glycémie, grâce à une baisse de la néoglucogénèse hépatique et une augmentation de la sensibilité à l'insuline de l'organisme (voir chapitre « obésité, diabète de type 2 et syndrome métabolique »), des inhibiteurs de 11βHSD1 peuvent éviter la progression vers les complications microvasculaires observées chez le diabétique. Cependant, le strict contrôle de la glycémie ne permet pas d'éviter entièrement le développement des complications microvasculaires, et rend nécessaire la découverte de nouveaux traitements permettant traiter plus globalement les patients diabétiques et dyslipidémiques (Girach et al. Int J Clin Pract 60(11) : 1471-1483, 2006 ; Taylor. Curr Diab Rep 8 (5) : 345-352 ; 2008). De manière intéressante, une étude de Chiodini et al. (Diabetes Care 30 : 83-88, 2007) a montré que la sécrétion de cortisol, chez les patients diabétiques, était directement associée à la présence de complications macro- ou micro-vasculaires chroniques. Par ailleurs, la réactivité microvasculaire et la fonction endothéliale sont altérées chez le patient souffrant du syndrome de Cushing, présentant un hypercortisolisme (Prazny et al. Physiol Rev 57 : 13-22, 2008).

Plus particulièrement, Bhatia et al. (Ann Ophtalmol 15 :128-130 ; 1983) ont montré une association entre les taux de cortisol plasmatiques élevés et la rétinopathie chez le diabétique.

Koh et al. ont montré que le traitement de patients atteints du syndrome de Cushing par adrénalectomie, permettant de réverser l'hypercortisolisme, améliore la fonction rénale.

Les paramètres cliniques de polyneuropathies (perception sensorielle, neuropathie autonome cardiaque) sont associés à une augmentation de la sécrétion de cortisol chez les patients diabétiques (Tsigos et al. J Clin Endocrinol Metab 76 :554-558, 1993).

Tous ces éléments montrent qu'une diminution de l'impact du cortisol par une inhibition locale de sa régénération, via des inhibiteurs de 11βHSD1, pourrait avoir un rôle favorable dans les troubles de la microcirculation associés au diabète (polyneuropathie, rétinopathie, et la néphropathie).

### B. Cognition et démence

Les troubles cognitifs légers sont des phénomènes communs aux personnes âgées et aux diabétiques de type 1 et 2, et peuvent conduire progressivement à la dépression ou la démence (Messier et al., Neurobiol. aging 26, 26; Greenwood et al.(2005), Neurobiol. aging 26, 45 (2005)). Autant dans le cas d'animaux que d'humains âgés, les différences inter-individuelles pour les fonctions cognitives générales ont été reliées aux différences d'exposition à long terme aux glucocorticoïdes (Lupien et al., Nat. Neurosci. 1, 69, (1998)). Par ailleurs, la dérégulation de l'axe HPA (hypothalamo-hypophyso-surrénalien)) résultant dans l'exposition chronique aux glucocorticoïdes de certaines sous-régions du cerveau a été proposée comme contribuant au déclin des fonctions cognitives (Mc Ewen et al., Curr. Opin. Neurobiol. 5, 205, 1995). La 11βHSD1 est abondante dans le cerveau et est exprimée dans de nombreuses sous régions incluant l'hypothalamus, le cortex frontal et le cerebellum (Sandeep et al., Proc. Natl. Acad. Sci. 101, 6734 (2004)). Les souris déficientes en 11βHSD1 sont protégées contre les dysfonctionnements de l'hypothalamus associés aux glucocorticoïdes qui sont rattachés à la vieillesse (Yau et al., Proc. Natl. Acad. Sci. 98, 4716, (2001)). De plus, dans des études chez l'Homme, il a été montré que l'administration de la carbenoxolone améliore la fluidité verbale et la mémoire verbale chez les personnes âgées (Yau et al., Proc. Natl. Acad. Sci. 98, 4716 (2001), Sandeep et al., Proc. Natl. Acad. Sci. 101, 6734 (2004)). Finalement, l'utilisation d'inhibiteurs sélectifs de la 11βHSD1 de type triazole a montré qu'ils prolongeaient la rétention de la mémoire chez des souris âgées (Rocha et al., Abstract 231 ACS meeting, Atlanta, 26-30 Mars 2006). Par ailleurs, il a été montré dans des modèles de rongeurs diabétiques que le taux de corticosterone contribuait au développement des pathologies cognitives induites par le diabète (Stranhan et al., Nature neurosc. 11,309 (2008 )). Ainsi, des inhibiteurs de la 11βHSD1 permettant une réduction de la régénération du cortisol au niveau de l'hippocampe, pourraient avoir un rôle bénéfique sur les fonctions cognitives chez les patients diabétiques âgés (Sandeep et al., Proc. Natl. Acad. Sci. 101, 6734 (2004)).

### C. Pression intra-oculaire

Les glucocorticoïdes peuvent être utilisés par voies topique ou systémique pour une grande variété de pathologies de l'ophtalmologie clinique. Une complication particulière de ces traitements est le glaucome induit par l'utilisation de corticostéroïdes. Cette pathologie est caractérisée par l'augmentation de la pression intra-oculaire (PIO). Dans les cas les plus graves et pour les formes non traitées, la PIO peut conduire à une perte de champ de vision partielle et éventuellement à une perte totale de la vision. La PIO est le résultat d'un déséquilibre entre la production d'humeur aqueuse et son drainage. L'humeur aqueuse est produite dans les cellules épithéliales non-pigmentées et le drainage est réalisé au travers des cellules du réseau trabéculaire. La 11βHSD1 est localisée dans les cellules épithéliales non-pigmentées et sa fonction est clairement l'amplification de l'activité des glucocorticoïdes dans ces cellules (Stokes et al., Invest. Ophthalmol. Vis. Sci. 41, 1629 (2000)). Cette notion est confirmée par l'observation que la concentration en cortisol libre est fortement excédentaire par rapport à la cortisone dans l'humeur aqueuse (ratio 14/1). L'activité fonctionnelle de la 11βHSD1 dans les yeux a été évaluée en étudiant l'action de la carbenoxolone chez des volontaires sains. Après sept jours de traitement à la carbenoxolone, la PIO est réduite de 18% (Rauz et al., Invest. Ophtamol. Vis. Sci. 42, 2037 (2001)). L'inhibition de la 11βHSD1 dans les yeux est donc prédite comme réduisant la concentration locale en glucocorticoïdes et la PIO, produisant un effet bénéfique dans le traitement du glaucome et d'autres désordres de la vision.

### D. Hypertension

Les substances hypertensives issues des adipocytes comme la leptine et l'angiotensinogène ont été proposées comme étant des éléments clés dans les pathologies d'hypertension reliées à l'obésité (Wajchenberg et al., Endocr. Rev. 21, 697 (2000)). La leptine qui est secrétée en excès chez les souris aP2-11βHSD1 transgéniques (Masuzaki et al., J. Clinical Invest. 112, 83 (2003)), peut activer différents réseaux de systèmes neuronaux sympathiques, incluant ceux qui régulent la pression artérielle (Matsuzawa et al., Acad. Sci. 892, 146 (1999)). De plus, le système rénine-angiotensine (SRA) a été identifié comme étant une voie déterminante dans la variation de la pression artérielle. L'angiotensinogène, qui est produit dans le foie et le tissu adipeux, est un substrat-clé pour la rénine et est à l'origine de l'activation du SRA. Le niveau plasmatique en angiotensiogène est significativement élevé dans les souris aP2-11βHSD1 transgéniques, comme le sont ceux de l'angiotensine II et de l'aldostérone (Masuzaki et al., J. Clinical Invest. 112, 83 (2003)) ; ces éléments conduisent à l'élévation de la pression artérielle. Le traitement de ces souris par de faibles doses d'un antagoniste du récepteur de l'angiotensine II abolit cette hypertension (Masuzaki et al., J. Clinical Invest. 112, 83 (2003)). Ces informations illustrent l'importance de l'activation locale des glucocorticoïdes dans le tissu adipeux et le foie, et suggère que cette hypertension puisse être causée ou exacerbée par l'activité de la 11βHSD1 dans ces tissus. L'inhibition de la 11βHSD1 et la réduction du niveau de glucocorticoïdes dans le tissu adipeux et/ou dans le foie est donc prédit comme ayant un rôle bénéfique pour le traitement de l'hypertension et des pathologies cardiovasculaires associées.

### D2. Hypertension artérielle sensible au sel

On estime qu'environ 30 à 50% de la population générale présente une sensibilité particulière au sel. De nombreuses évidences suggèrent un lien entre la sensibilité au sel et l'hypertension artérielle et les risques cardiovasculaires (Weinberger MH, Curr Opin Cardiol 2004; 19 :353-356). Il a été montré que les sujets sensibles au sel présentent une variabilité de la fréquence cardiaque diminuée, ainsi qu'une pression artérielle et une production de cortisol augmentée durant un stress mental, comparativement à des sujets non sensibles (Weber CS et al., Journal of Human Hypertension 2008; 22:423-431). Par ailleurs, une récente étude de Liu Y et al. (Physiol Genomics 2008 Sept 30) a démontré chez le rat sensible au sel de Dahl (Dahl salt-sensitive rat), que l'inhibition spécifique de l'expression de la 11βHSD1 médullaire rénale, par l'utilisation de shRNA, permet de diminuer très nettement chez les animaux l'élévation de la pression artérielle moyenne induite par un régime salé. Ces éléments permettent de penser qu'un inhibiteur de l'enzyme 11βHSD1 aurait très probablement un effet bénéfique sur cette forme d'hypertension artérielle.

### E. Ostéoporose

Le développement du squelette et les fonctions osseuses sont aussi régulées par l'action des glucocorticoïdes. La 11βHSD1 est présente dans les ostéoclastes et ostéoblastes. Le traitement de volontaires sains par la carbenoxolone a montré une diminution des marqueurs de résorption osseuse sans changement dans les marqueurs de formation des os (Cooper et al., Bone, 27, 375 (2000)). L'inhibition de la 11βHSD1 et la réduction du niveau de glucocorticoïdes dans les os pourraient donc être utilisées comme un mécanisme de protection dans le traitement de l'ostéoporose.

### F. Lipodystrophie associée à une thérapie antirétrovirale hautement active (HAART), ou HAL syndrome.

L'utilisation d'un traitement antiretroviral intensif pour les patients atteints du sida induit souvent un syndrome de lipodystrophie (HAL) ressemblant au syndrome de Cushing, et associant augmentation de la masse grasse abdominale, hypertriglycéridémie et résistance à l'insuline. Il a été montré (Sutinen et al., Diabetologia, 47,1668 (2004)) que cette lipodystrophie (HAL) était associée à une augmentation de l'expression de la 11βHSD1 dans le tissu adipeux des patients. Des inhibiteurs de la 11βHSD1, permettant une réduction de la régénération du cortisol au niveau du tissu adipeux, pourraient donc avoir un rôle bénéfique chez les patients atteints de lipodystrophie associée à un traitement intensif du sida par des antitretroviraux (HAL syndrome).

### G. Maladies infectieuses

Certaines infections, comme la tuberculose, sont associées à des dérèglements de la réponse immunitaire (Ellner JJ, J. Lab. Clin. Med, 130, 469, (1997)). Cette particularité, qui s'accompagne le plus souvent de l'augmentation de la sécrétion de certaines cytokines (IL-10, TNFα) et/ou de la réponse à certaines cytokines, semble trouver, au moins en partie, sa cause dans l'exposition tissulaire locale des cellules immunitaires aux glucocorticoïdes. Par ailleurs, l'administration de glucocorticoïdes de synthèse chez l'homme ou l'animal provoque la réactivation de la tuberculose chez l'homme et chez l'animal (Haanas OC et al. Eur. J. Repir. Dis. 64, 294 (1998), Brown et al. Infect. Immun. 63, 2243, (1995)). De même, les stress divers, activateurs de l'axe HPA, ont pour conséquence une réactivation de cette infection.

En dehors de ces cas particuliers, les taux circulants de glucocorticoïdes ainsi que l'activation de l'axe HPA, semblent être normaux chez des patients atteints de tuberculose (Baker et al. Am. J Resp.Crit. Care. Med., 162, 1641 (2000)). Par contre, les taux de cortisol versus cortisone dans le liquide bronchoalvéolaire, semblent augmentés, traduisant une modulation du métabolisme des glucocorticoïdes vers la forme active (notamment dépendante de l'activité de la 11βHSD1). L'inhibition de la 11βHSD1 au niveau des tissus périphérique et notamment des poumons, pourrait par conséquent produire un effet bénéfique sur la stabilisation puis sa reversion de l'infection.

### H. Hypertrophie cardiaque et insuffisance cardiaque

Les maladies cardiovasculaires représentent la première cause de morbidité et de mortalité dans les pays industrialisés, et l'hypertrophie ventriculaire gauche (HVG) est un facteur de risque indépendant de mortalité cardiovasculaire (Havranek EP, Am J Med 121 :870-875, 2008). En dehors des causes génétiques, des conditions pathologiques telles que l'hypertension artérielle, l'infarctus du myocarde, ou l'insuffisance rénale, peuvent conduire à une hypertrophie compensatoire, progressant par la suite vers une insuffisance cardiaque chronique. L'activité 11βHSD1, permettant la conversion de 11-déshydrocorticostérone en corticostérone, est exprimée dans les cardiomyocytes de rats nouveaux-nés, et contribue à l'activité modulatrice des glucocorticoïdes et de l'aldostérone dans le coeur (Sheppard and Autelitano, Endocrinology 143:198-204, 2002). En utilisant ces cellules, Lister et al. (Cardiovascular Research 70 : 555-565, 2006) ont montré que l'hypertrophie des cardiomyocytes, induite par des agents pharmacologiques, s'accompagnait d'une augmentation de l'activité de l'enzyme 11βHSD1. Dans cette même étude, l'utilisation du RU-486, antagoniste spécifique des récepteurs aux glucocorticoïdes, permettait de diminuer l'hypertrophie des cellules.

Des inhibiteurs de l'activité 11βHSD1 pourraient donc limiter l'hypertrophie cardiaque et ainsi d'éviter la progression vers l'insuffisance cardiaque.

### I. Maladies hépatiques :

### II. Stéatose hépatique :

Des études chez des obèses sévères (BMI > 35 kg/m2) rapportent une prévalence de 91 % pour la stéatose et de 37 % pour la stéatohépatite *(*Neuschwander-Tetri & Caldwell, Hepatology, 37, 1202 - 1219, 2003*).* Le diabète de type 2 est un autre facteur majeur associé à la stéatose avec une prévalence de 70 % rapportée sur un échantillon de 3000 diabétiques italiens (Targher et al., Diabetes Care , 30, 1212 - 1218, 2007). Par ailleurs, il a été observé une association entre l'insulinorésistance et la stéatose hépatique indépendamment de l'obésité chez les patients atteints de stéatose hépatique non alcoolique (Manchesini et al., Diabetes, 50, 1844 - 1850, 2001). Chez les obèses, l'activité 11βHSD1 paraît modifiée ainsi qu'en témoigne l'activation de la cortisone administrée oralement, l'excrétion urinaire des métabolites du cortisol ou l'expression hépatique tissulaire de la 11βHSD1. (Tomlinson et al., Endocrine Rev, 25, 831 - 866, 2004 *;* Rask et al., J. Clin. Endocrin. Metab., 86, 1418 - 1421, 2001 *;* Stewart et al., J. Clin. Endocrin. Metabol. 84, 1022-1027, 1999 ; Valsamakis et al., J. Clin. Endocrinol. Metabol., 89, 4755 - 4761, 2004). Les souris transgéniques surexprimant 11βHSD1 au niveau du tissu adipeux ou au niveau hépatique développent une stéatose hépatique et une dyslipidémie (Masuzaki et al., Sciences 294, 2166 - 2170, 2001 *;* Paterson et al., PNAS, 101, 7088 - 7093, 2004). L'inhibition de la 11βHSD1 chez le rat réduit la triglycéridémie à jeûn suite à une diminution de la sécrétion des triglycérides hépatiques et à une augmentation de la capture et de l'oxydation tissulaire des acides gras, qui se traduit également au niveau hépatique par une réduction significative des triglycérides (Berthiaume et al., Am. J. Physiol. Endocrinol. Metab., 293, 1045 - 1052, 2007). La réduction locale de glucocorticoïde actif par inhibition de l'activité 11βHSD1 est donc envisagée pour diminuer les effets insulinorésistants et lipidiques des glucocorticoïdes et ainsi diminuer la stéatose hépatique.

### I2. Stéatohépatite Métabolique :

La stéatohépatite métabolique représente un stade d'évolution de la stéatose hépatique métabolique chez certaines personnes. Une corrélation est décrite entre le cortisol urinaire, la concentration en cortisol post-dexaméthasone et le grade de nécroinflammation et de fibrose hépatique chez des sujets atteints de stéatohépatite métabolique suggérant l'existence d'un hypercorticolisme subclinique ou locale (Targher et al., Clin. Endocrinol., 64, 337 - 341, 2006). Une correction générale et locale (au niveau centrolobulaire) de l'insulinorésistance ainsi qu'une amélioration de l'oxydation des acides gras hépatiques par inhibition de l'activité 11βHSD1, et aussi la réduction des effets pro-fibrotiques du cortisol sont donc prédictifs d'une amélioration de l'évolution pathologique.

### I3. Régénération hépatique :

Le foie présente une capacité de régénération importante, tout à fait nécessaire en cas d'agressions d'origines infectieuses ou non, en particulier provenant du tractus digestif. Par exemple une apoptose ou une nécrose hépatique peuvent résulter d'une toxicité médicamenteuse, virale, alcoolique, métabolique, cholestatique ou ischémique vasculaire. Les glucocorticoïdes inhibent la prolifération hépatocytaire et la régénération tissulaire hépatique (Tsukamoto & Kojo, Gut, 30, 387 - 390, 1989 *;* Nagy et al., Hepatology, 28, 423 - 429, 1998 *;* Tannuri et al., Pediatr. Transplantation, 12, 73-79, 2008). Une inhibition de l'activité réductase 11βHSD1 pourrait dans ce contexte diminuer les effets locaux négatifs du cortisol sur la régénération hépatique et sont à mettre en ligne avec les effets pro-angiogéniques de ces inhibiteurs et avec leur action positive sur certains facteurs de croissance.

### J. Cicatrisation des plaies cutanées chroniques :

La cicatrisation des plaies chroniques dépend du contexte pathologique sous-jacent qui modifie et désynchronise les étapes physiologiques de la cicatrisation. Dans l'ulcère chronique du diabétique, l'intérêt potentiel des inhibiteurs de 11βHSD1 doit se voir à la fois dans la correction des manifestations du diabète, en tenant compte du rôle pathologique local des corticoïdes endogènes au niveau de la plaie et de l'état d'avancement pathologique. Il existe un certain nombre d'évidences montrant que les corticoïdes endogènes sont directement impliqués dans l'altération de la cicatrisation des plaies chez l'homme et dans des modèles animaux rongeurs (Goforth et al., J. Foot Surgery, 19, 199 - 2002, 1980 *;* Dostal et al, Arch. Surg, 125, 636 -640, 1990 *;* Bitard, Am. J. Pathology, 152, 547 - 554, 1998). Une production locale de cortisol est prédite par la présence d'une activité réductase 11βHSD1 au niveau endothélial, fibroblastique, cutané chez l'homme et chez les rongeurs (Gong et al., Steroids 73, 1187 -1196, 2008 *; ;* Hammami et al., J. Clin. Endocrinol. Metabol., 73, 326 - 334, 1991 *;* Cooper et al., ENDO 2003 *;* Teelucksingh et al., Lancet, 335, 1060 - 1063, 1990). Le cortisol et autres glucocorticoïdes inhibent la cicatrisation de l'ulcère cutané par de nombreux mécanismes et à différents étapes : altération de la vasomotricité microcirculatoire, inhibition de la phase inflammatoire en particulier sur la synthèse de prostaglandines, de leukotriènes, de cytokines, comme TNFalpha et les productions de IL-1beta, IL-4.... et la signalisation de IFNgamma, augmentation de l'infection, réduction de la motilité cellulaire et de la prolifération des kératinocytes, réduction de l'expression de facteurs pro-angiogéniques comme VEGF, suppression de l'expression de TGFbeta 1 et 2 essentiels dans la production de collagène par les fibroblastes et leur transformation en myofibroblastes, suppression de l'expression de MMP1, 2 , 9 et 10 et induction de TIMP bloquant ainsi le remodelage, promotion de la différentiation terminale épidermique mais inhibition des premiers stades de différentiation, avec pour conséquence une fragilisation de l'épiderme (Bitard, Am. J. Pathology, 152, 547 - 554, 1998 , Beer et al., Vitam. Horm., 59, 217 - 239, 2000 *;* Rosen & Miner, Endocrine Review, 26, 452 - 464, 2005*,* Stojadinovic et al., J. Biol. Chem , 282, 4021 - 4034, 2007*).* A l'inverse et comme attendu, une inhibition de l'activité réductase 11βHSD1 est décrite pour induire une vasodilatation, un effet pro-angiogénique et anti-infectieux (voir les chapitres correspondants) et dans certaines situations inflammatoires pour produire une exacerbation et une surexpression de facteur de croissance tel que TGFbeta (Zhang et al., J. Immunology, 179, 6325 - 6335, 2007). Des inhibiteurs de 11βHSD1 devraient donc permettre, en agisant ainsi, d'améliorer la cicatrisation des plaies cutanées chroniques.

On a maintenant trouvé des dérivés d'urée de tétrahydroquinoxaline, portant un noyau adamantane, qui modulent l'activité de la 11βHSD1.

La présente invention a pour objet des composés répondant à la formule (I) : 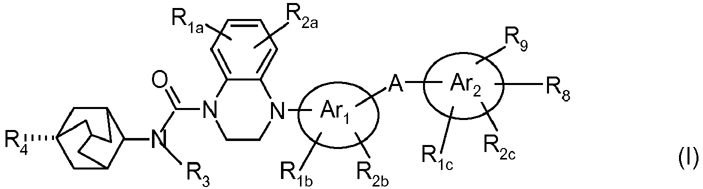 dans laquelle :
- A représente une liaison, un atome d'oxygène ou un groupe -O-CH₂-,
- Ar₁ représente un groupe phényle ou hétéroaryle,
- Ar₂ représente un groupe phényle, un groupe hétéroaryle ou un groupe hétérocycloalkyle,
- R_{1a,b,c} et R_{2a,b,c}, identiques ou différents, représentent chacun un atome d'hydrogène ou d'halogène ou un groupe alkyle ; cycloalkyle éventuellement substitué par un groupe alkyle, halogénoalkyle, alcoxy-alkyle, alcoxy-halogénoalkyle ou -COOR₅ ; -alkyl-cycloalkyle éventuellement substitué par un ou plusieurs atomes d'halogène ; -OR₅ (hydroxy ou alcoxy) ; hydroxy-alkyle ; alcoxy-alkyle ; alcoxy-alcoxy ; halogénoalkyle ; -O-halogénoalkyle ; oxo ; -CO-alkyle ; -CO-alkyl-NR₆R₇ ; -CO-halogénoalkyle ; -COOR₅ ; alkyl-COOR₅ ; -O-alkyl-COOR₅ ; -SO₂-alkyle ; -SO₂-cycloalkyle ; -SO₂-alkyl-cycloalkyle ; -SO₂-alkyl-OR₅ ; -SO₂-alkyl-COOR₅ ; -SO₂-alkyl-NR₆R₇; -SO₂-halogénoalkyle ; alkyl-SO₂-alkyle ; -SO₂-NR₆R₇; -SO₂-alkyl-alcoxy-alcoxy ; -CONR₆R₇ ; -alkyl-CONR₆R₇ ou -O-alkyl-NR₆R₇, ou encore R₁ₐ, R_{1b}, R_{1c} sont liés respectivement à R₂ₐ, R_{2b}, R_{2c} et à l'atome de carbone qui les porte et représentent -O-alkyl-O- ;
- R₃ représente un atome d'hydrogène ou un groupe alkyle,
- R₄ représente un atome d'hydrogène ou d'halogène ou un groupe cyano, -OR₅, hydroxy-alkyle, -COOR₅, -NR₆R₇, -CONR₆R₇, -SO₂-alkyle ou -SO₂-NR₆R₇, -NR₆-COOR₅, -NR₆-COR₅, -CO-NR₆-alkyl-OR₅ ;
- R₅, R₆ et R₇, identiques ou différents, représentent chacun un atome d'hydrogène, un groupe alkyle ou un groupe -alkyl-phényle, et
- R₈ représente un groupe alkyle, alkyl-Si(alkyle)₃ ; -SO₂-alkyl-Si(alkyle)₃ ; phényle ; alcoxy-imino ; alkyle-cycloalkyle éventuellement substitué par un ou plusieurs atomes d'halogène; hétérocycloalkyle substitué par un ou plusieurs atomes d'halogène, un ou plusieurs groupes hydroxy ou hydroxy-alkyle ; ou bien R₈ et R₉ ensemble avec l'atome de carbone auquels ils sont liés forment un groupe cycloalkyle éventuellement substitué par un ou plusieurs atomes d'halogène ou un ou plusieurs groupes carboxy ;
- R₉ représente un atome d'hydrogène ou un groupe alkyle ;
à la condition que lorque R₈ est un groupe alkyle, il est fixé sur l'atome de silicium d'un groupe Ar₂.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou d'acides ou être salifiés par des acides ou des bases, notamment des acides ou des bases pharmaceutiquement acceptables. De tels sels d'addition font partie de l'invention. Ces sels sont avantageusement préparés avec des acides ou des bases pharmaceutiquement acceptables, mais les sels d'autres acides ou bases utiles, par exemple, pour la purification ou l'isolement des composés de formule (I), font également partie de l'invention.

Les composés de formule (I) peuvent également exister sous forme d'hydrates ou de solvats, à savoir sous forme d'associations ou de combinaisons avec une ou plusieurs molécules de solvant. De tels solvats font également partie de l'invention.

Dans le cadre de la présente invention, et sauf mention différente dans le texte, on entend par:
- un atome d'halogène : un fluor, un chlore, un brome ou un iode ;
- un groupe alkyle : un groupe aliphatique saturé linéaire ou ramifié comportant de 1 à 5 atomes de carbone. A titre d'exemples, on peut citer les groupes méthyle, éthyle, propyle, méthylpropyle, isopropyle, butyle, isobutyle, tertbutyle ou pentyle ;
- un groupe cycloalkyle : un groupe alkyle cyclique comportant de 3 à 6 atomes de carbone. A titre d'exemples, on peut citer les groupes cyclopropyle, cyclobutyle, cyclopentyle,
- un groupe alcoxy : un radical de formule -O-alkyle, où le groupe alkyle est tel que défini précédemment ;
- un groupe hydroxy-alkyle : un radical de formule alkyle-OH, où le groupe alkyle est tel que défini précédemment ;
- un groupe alcoxy-alkyle : un radical de formule alkyle-O-alkyle, où les groupes alkyles, identiques ou différents, sont tels que définis précédemment. A titre d'exemples, on peut citer -(CH₂)₂-O-CH₃, -(CH₂)₃-O-CH₃, -CH-(CH₂-O-CH₃)₂ ;
- un groupe alcoxy-alcoxy : un radical de formule -O-alkyl-O-alkyle, où les groupes alkyles, identiques ou différents, sont tels que définis précédemment ;
- un groupe halogénoalkyle : un groupe alkyle tel que défini ci-dessus substitué par 1 à 5 atomes d'halogène, tels que définis précédemment. On citera par exemple le groupe trifluorométhyle ;
- un groupe hétéroaryle : un groupe aromatique comprenant de 5 à 9 atomes, dont 1 à 3 hétéroatomes, tels que l'azote, l'oxygène ou le soufre. On peut notamment citer les groupes pyridinyle, pyrimidinyle, pyridazinyle ou thiazolyle ; et
- un hétérocycloalkyle : un groupe alkyle mono-, bi-cyclique éventuellement ponté comportant de 4 à 9 atomes ou éventuellement partiellement insaturé et dont 1 ou 2 atomes sont des hétéroatomes, tels que l'oxygène, l'azote, le soufre ou le silicium. On peut notamment citer les groupes pyrrolidinyle, pipéridinyle, pipérazinyle, tétrahydropyranyle, morpholinyle, homopipérazinyle, 3,8-diazabicyclo[3.2.1]octane, thiomorpholinyle et thiomorpholinyl-1,1-dioxide, octahydro-pyrrolo[3,4-c]pyrrole, 1,2,3,6-tetrahydro-pyridine, 2,5-diaza-bicyclo[2.2.1]heptane, azasilinane ;
- une fonction carbonyle est représentée par CO.

Dans le cadre de la présente invention, R_{1a,b,c} désigne les groupes R₁ₐ, R_{1b} et R_{1c} et R_{2a,b,c} désigne les groupes R₂ₐ, R_{2b} et R_{2c}. Lorsque Ar₂ représente un groupe hétérocycloalkyle, les groupes R_{1c}, R_{2c} R₈ et R₉ peuvent être portés par n'importe quel atome dudit hétérocycle, qu'il s'agisse d'un atome de carbone ou d'un hétéroatome (par exemple un atome d'azote), y compris par le même atome dudit hétérocycloalkyle (par exemple lorsqu'il s'agit d'un atome de soufre).

Dans les composés de formule (I) selon l'invention, le groupe R₄ et le groupe urée peuvent être en position trans ou en position cis. Les composés de formule (I) dans lesquels R₄ et le groupe urée sont en position trans sont particulièrement préférés.

Parmi les composés de formule (I) selon l'invention, on peut citer un sous-groupe de composés dans lesquels A représente une liaison.

Un autre sous-groupe de composés de formule (I) selon l'invention est tel que Ar₁ représente un groupe hétéroaryle. Avantageusement, Ar₁ représente un groupe pyridinyle.

Un autre sous-groupe de composés de formule (I) selon l'invention est tel que Ar₂ représente un groupe hétérocycloalkyle. Avantageusement, Ar₂ représente un groupe pipéridinyle, pipérazinyle ou azasilinanyle.

Parmi les composés de formule (I) selon l'invention dans lesquels Ar₁ représente un groupe phényle ou un hétéroaryle à 6 chaînons, on peut citer ceux dans lesquels la liaison entre les noyaux A-Ar₂ et Ar₁ est en position para par rapport à la liaison entre Ar₁ et l'atome d'azote du noyau tétrahydroquinoxaline auquel il est lié.

Parmi les composés de formule (I) selon l'invention dans lesquels Ar₂ représente un groupe hétéroaryle ou hétérocycloalkyle, on peut citer ceux qui sont liés au groupe A par un hétéroatome.

Un autre sous-groupe de composés de formule (I) selon l'invention est tel que R_{1a,b,c} et R_{2a,b,c}, représentent chacun un atome d'hydrogène.

Un autre sous-groupe de composés de formule (I) selon l'invention est tel que R₃ représente un atome d'hydrogène.

Un autre sous-groupe de composés de formule (I) selon l'invention est tel que R₄ représente un groupe hydroxy-alkyle ou -CONH₂.

Un autre sous-groupe de composés de formule (I) selon l'invention est tel que R₈ représente un groupe alkyle, alkyl-Si(alkyle)₃ ; -SO₂-alkyl-Si(alkyle)₃ ; phényle ; alcoxy-imino ; hétérocycloalkyle substitué par un ou plusieurs atomes d'halogène, un ou plusieurs groupes hydroxy ou hydroxy-alkyle ; ou bien R₈ et R₉ ensemble avec l'atome de carbone auquels ils sont liés forment un groupe cycloalkyle éventuellement substitué par un ou plusieurs atomes d'halogène ou un ou plusieurs groupes carboxy ;
- R₉ représente un atome d'hydrogène ou un groupe alkyle ;
à la condition que lorque R₈ est un groupe alkyle, il est fixé sur l'atome de silicium de Ar₂.

Les sous-groupes définis ci-dessus pris séparément ou en combinaison font également partie de l'invention.

Un groupe de composés de formule (I) particulièrement préférés au sens de l'invention est constitué des composés de formules (I) dans laquelle :
- A est une liaison directe ;
- Ar₁ est une hétéroaryle ;
- Ar₂ est un hétérocycloalkyle ;
- R₃ représente un atome d'hydrogène,
- R₄ représente un groupe OH ou -CONH₂,
   R₈ représente un groupe alkyle, alkyl-Si(alkyle)₃ ; -SO₂-alkyl-Si(alkyle)₃ ; phényle ; alcoxy-imino ; hétérocycloalkyle substitué par un ou plusieurs atomes d'halogène, un ou plusieurs groupes hydroxy ou hydroxy-alkyle ; ou bien R₈ et R₉ ensemble avec l'atome de carbone auquels ils sont liés forment un groupe cycloalkyle éventuellement substitué par un ou plusieurs atomes d'halogène ou un ou plusieurs groupes carboxy ;
- R₉ représente un atome d'hydrogène ou un groupe alkyle ;
à la condition que lorque R₈ est un groupe alkyle, il est fixé sur l'atome de silicium de Ar₂.

Avantageusement, R₈ représente un groupe alkyle, alkyle-Si(alkyle)₃ ; -SO₂-alkyle-Si(alkyle)₃ ; phényle ; alcoxy-imino ; pyrrolidinyle substitué par un ou plusieurs atomes d'halogène, un groupe hydroxy ou hydroxy-alkyle ; thiomorpholinyle ; ou bien R₈ et R₉ ensemble avec l'atome de carbone auquels ils sont liés forment un groupe cycloalkyle éventuellement substitué par un ou plusieurs atomes d'halogène ou un ou plusieurs groupes carboxy ; R₉ représente un atome d'hydrogène ou un groupe alkyle à la condition que lorque R₈ est un groupe alkyle, il est fixé sur l'atome de silicium de Ar₂.

Parmi les composés de formule (I) objets de l'invention, on peut notamment citer les composés suivants :
Acide Trans 4-[5-(4-Trimethylsilanylmethyl-piperazin-1-yl)-pyridin-2-yl]-3,4-dihydro-2H-quinoxaline-1-carboxylique (5-hydroxy-adamantan-2-yl)-amide ;
Acide Trans 4-(4-tert-Butoxyimino-3,4,5,6-tetrahydro-2H-[1,3']bipyridinyl-6'-yl)-3,4-dihydro-2H-quinoxaline-1-carboxylique (5-hydroxy-adamantan-2-yl)-amide ;
Acide Trans 4-{5-[4-(2-Trimethylsilanyl-ethanesulfonyl)-piperazin-1-yl]-pyridin-2-yl}-3,4-dihydro-2H-quinoxaline-1-carboxylique (5-hydroxy-adamantan-2-yl)-amide ;
Acide Trans 4-{5-[4-(2,2-Difluoro-cyclopropylmethyl)-piperazin-1-yl]-pyridin-2-yl}-3,4-dihydro-2H-quinoxaline-1-carboxylique (5-carbamoyl-adamantan-2-yl)-amide ;
Acide Trans 4-[4-(1,1-Dioxo-1lambda6-thiomorpholin-4-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans4-[4-((R)-2-Hydroxymethyl-pyrrolidin-1-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans 4-[4-((S)-2-Hydroxymethyl-pyrrolidin-1-yl)-3,4,5,6-tetrahydro-2H-[1,3']bipyridinyl-6'-yl]-3,4-dihydro-2H-quinoxaline-1 -carboxylique (5-carbamoyl-adamantan-2-yl)-amide ;
Acide Trans4-[4-((S)-3-Hydroxy-pyrrolidin-1-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans 4-[4-((R)-3-Hydroxy-pyrrolidin-1-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans 4-[4-(3,3-Difluoro-pyrrolidin-1-yl)-3,4,5,6-tetrahydro-2H-[1,3']bipyridinyl-6'-yl]-3,4-dihydro-2H-quinoxaline-1 -carboxylique (5-carbamoyl-adamantan-2-yl)-amide ;
Acide Trans 4-[5-(1,1-Difluoro-6-aza-spiro[2.5]oct-6-yl)-pyridin-2-yl]-3,4-dihydro-2H-quinoxaline-1-carboxylique (5-carbamoyl-adamantan-2-yl)-amide ;
Acide Trans 4-[5-(4-Methyl-4-phenyl-[1,4]azasilinan-1-yl)-pyridin-2-yl]-3,4-dihydro-2H-quinoxaline-1-carboxylique (5-carbamoyl-adamantan-2-yl)-amide ;
Acide Trans 4-[5-(4,4-Dimethyl-[1,4]azasilinan-1-yl)-pyridin-2-yl]-3,4-dihydro-2H-quinoxaline-1-carboxylique (5-carbamoyl-adamantan-2-yl)-amide.

Il est à noter que les composés ci-dessus ont été nommés en nomenclature IUPAC par l'intermédiaire du logiciel AutoNom (Beilstein Informations system).

Dans ce qui suit, on entend par groupe protecteur (GP) un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 3rd Edition (John Wiley & Sons, Inc., New York).

On entend par groupe partant (Lg, E, V, X, Z), dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, paranitrophényle, etc. Des exemples de groupes partants ainsi que des méthodes pour leur préparation sont donnés dans « Advances in Organic Chemistry », J. March, 3rd Edition, Wiley Interscience, p. 310-316.

Conformément à l'invention, on peut préparer les composés de formule générale (I) selon les procédés ci-après.

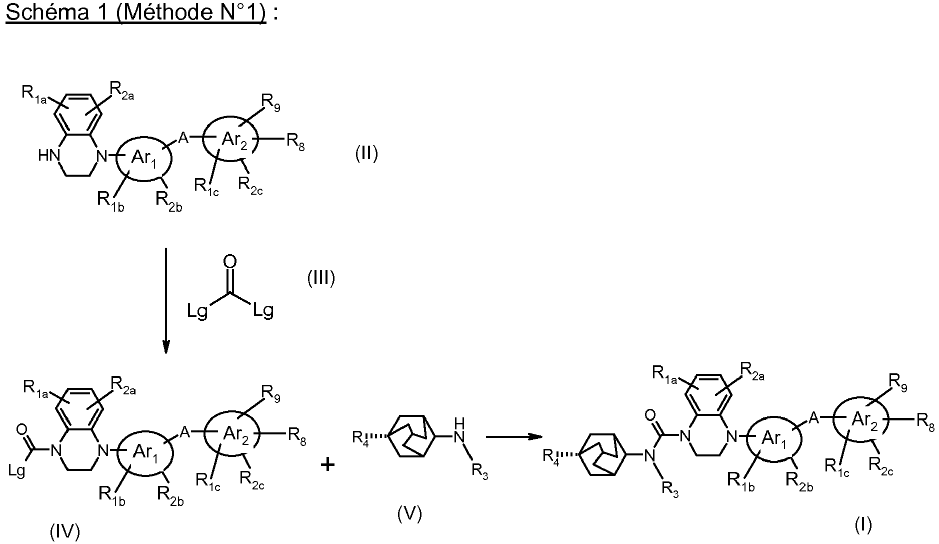

Dans le schéma 1, les composés de formule (IV) peuvent être préparés par réaction entre les intermédiaires de formule (II) et un carbonyle de formule (III) présentant deux groupes partant Lg (par exemple un atome de chlore, un groupe trichlorométhoxy, un groupe para-nitrophényle, un groupe imidazole ou méthyl-imidazolium) en présence d'une base comme la triéthylamine ou la diisopropylamine, dans un solvant tel que le dichlométhane ou le tétrahydrofurane et à une température variant de la température ambiante à 80°C. Les composés de formule (I) sont ensuite obtenus par couplage entre les dérivés activés (IV) et les amines (V) en présence ou non d'une base comme la triéthylamine ou le carbonate de potassium, dans un solvant tel que le tétrahydrofurane, le dichlorométhane, l'acétonitrile, le diméthylformamide ou l'eau, à une température variant de la température ambiante à 100°C.

Dans certains cas, quand R₁ ou R₂ est un alcool, ou R₁, R₂ ou R₄ est une amine primaire ou secondaire ou un acide ou un bioisostère de fonction acide (tétrazole,...) ou si Ar₁ ou Ar₂ présente dans le composé (I) une fonction amine secondaire, il est alors nécessaire de réaliser la Méthode N°1 avec un dérivé (II) ou (V) où les fonctions précédemment citées sont rendues non réactives par la présence d'un groupe protecteur (par exemple, pour une amine : un groupe Boc, Bn ou CBz ; pour un alcool ; un groupe Bn ; pour un acide : un groupe ester ; pour un tétrazole : un groupe benzyle). Finalement, pour obtenir la fonctionnalité désirée, il faut ensuite réaliser une réaction de déprotection dans des conditions connues par l'homme de métier.

Les hétérocycles de formule générale (V) sont disponibles commercialement ou peuvent être préparés par des méthodes décrites dans la littérature (par exemple WO 2007/077949, US 2005/0215784 A1, US 2005/0245745 A1, Journal of Organic Chemistry (2005), 70(20), 7919-7924).

Le schéma 2 détaille une synthèse des composés de formule (II).

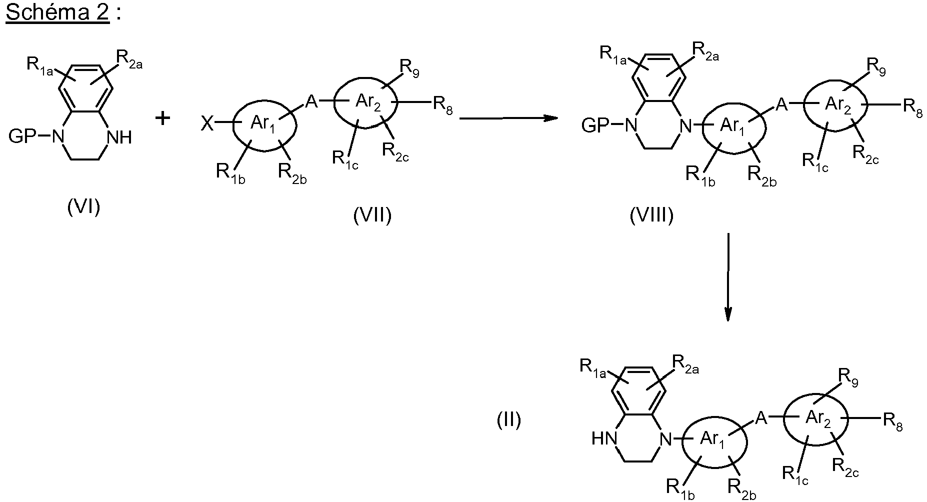

Dans le schéma 2, les composés de formule (VIII) peuvent être préparés par couplage entre une tétrahydro-quinoxaline monoprotégée de formule (VI) et un dérivé (VII) présentant un groupe partant X (par exemple un halogène, un groupe tosylate, triflate ou nanoflate) en présence d'un catalyseur organométallique tel qu'un dérivé du palladium, en présence ou non d'une phosphine telle que la tritertbutylphosphine ou la triphénylphosphine, en présence d'une base comme le carbonate de potassium, le fluorure de potassium, le tertbutylate de potassium ou le phosphate de potassium dans un solvant ou mélange de solvants tel que le dioxane, l'éthylène glycol diméthyléther, le toluène, le tétrahydrofurane ou l'eau, à une température variant de la température ambiante à 100°C. Les amines (II) sont obtenues par déprotection de la fonction amine des composés de formule (VIII), par des méthodes choisies parmi celles connues de l'homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Les hétérocycles de formule générale (VI) sont disponibles commercialement ou peuvent être préparés par des méthodes décrites dans la littérature (par exemple «Comprehensive heterocyclic chemistry», Katritzky et al., 2rd Edition (Pergamon press) ; Krchnak, V. et al., Tet. Lett (2001), 42, 2443-2446 ; Eary, C. T. et coll., Tet. Lett. (2006), 47, 6899-6902 ; Savrides, E.-M. et al., J. Het. Chem. (2005), 42, 1031-1034. De Selms, R.C. et al., J. Het. Chem. (1974), 11(4), 595-7.

Les composés de formule générale (VII) sont disponibles commercialement ou peuvent être préparés par des méthodes décrites dans la littérature (par exemple Z. Sui et coll., Bioorg. Med. Chem. Lett. (2003), 13, 761-765 ; Chopa, A. B. et coll., J. Organomet. Chem. (2005), 690(17), 3865-3877 ; Düggeli, M. et coll., Org. Biomol. Chem. (2003), 1(11), 1894-1899 ; Gros, P. et coll., J. Org. Chem (2003), 68(5), 2028-2029 ; Bouillon, A. et coll., Tet. (2002), 58(14), 2885-2890 ; Balle, T. et coll., J. Med. Chem. (2006), 49(11), 3159-3171 ; M. A. Ismail et coll., J. Med. Chem. (2006), 49(17), 5324-5332, Gu, Y. G. et coll., J. Med. Chem. (2006), 49(13), 3770-3773 ; Serafin, B. et coll., Eur. J. Med. Chem. (1977), 12(4), 325-31 ; Schmidt, H.-W. et coll., J. Het. Chem. (1987), 24(5), 1305-7 ; Walsh, D. A. et coll., J. Med. Chem. (1990), 33(7), 2028-32 ; WO 2005/042521; EP 0 277 725).

Le schéma 3 détaille une synthèse des composés de formule (VIII) dans lesquels Ar₁ représente un noyau pyridine (Y=C) ou pyrimidine (Y=N) ; ces composés seront appelés ci-après composés de formule (IX).

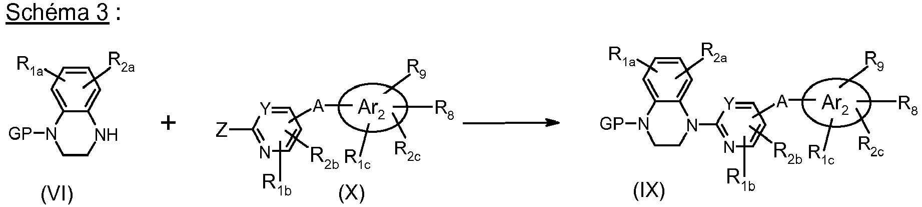

Dans le schéma 3, les composés de formule (IX) peuvent être préparés par une réaction de substitution nucléophile aromatique entre une tétrahydro-quinoxaline monoprotégée de formule (VI) et un dérivé (X) présentant un groupe partant Z (par exemple un halogène ou un groupe alkylsulfonyle) en présence d'une base comme le sel de lithium de l'hexaméthyldisilazane ou l'hydrure de sodium dans un solvant tel que le tétrahydrofurane, la N-méthylpyrrolidinone, le diméthylsulfoxyde ou le diméthylformamide, à une température variant de la température ambiante à 100°C.

Le schéma 4 détaille une synthèse des composés de formule (VIII) dans lesquels Ar₁ représente un noyau phényle et A représente une liaison ; ces composés seront appelés ci-après composés de formule (XI).

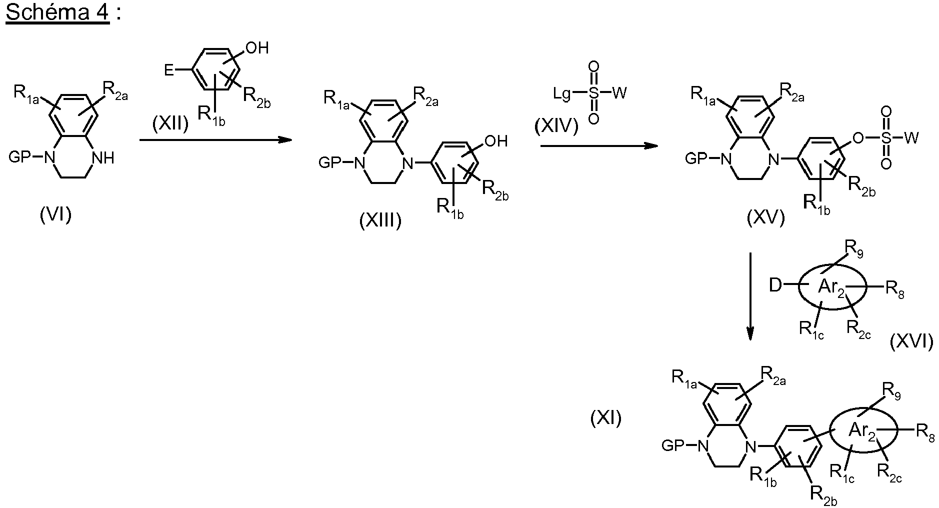

Dans le schéma 4, les composés de formule (XIII) peuvent être préparés par une réaction de couplage entre une tétrahydro-quinoxaline monoprotégée de formule (VI) et un dérivé (XII) présentant un groupe partant E (par exemple un halogène, triflate ou nanoflate) en présence d'un catalyseur organométallique tel qu'un dérivé du palladium, en présence ou non d'une phosphine telle que la tritertbutylphosphine ou la triphénylphosphine, en présence d'une base comme le carbonate de potassium ou cesium, le fluorure de potassium, le tertbutylate de potassium ou le phosphate de potassium dans un solvant ou mélange de solvants tel que le dioxane, l'éthylène glycol diméthyléther, le toluène, le tétrahydrofurane ou l'eau, à une température variant de la température ambiante à 100°C. La fonction phénol est ensuite transformée en ester sulfonique pour former les composés (XV) par action d'un dérivé sulfonique (XIV), où SO₂W représente par exemple un groupe mésylate, tosylate, triflate ou nanoflate, tel qu'un anhydride sulfonique (Lg=OSO₂W), un fluorure d'acide sulfonique (Lg=F) ou un chlorure d'acide sulfonique (Lg=Cl), en présence d'une base ou d'un mélange de bases comme la triéthylamine, la pyridine, la diméthylaminopyridine, la diisopropyléthylamine ou le carbonate de potassium dans un solvant ou mélange de solvants tel que le dichlorométhane, le chloroforme, le toluène, le tétrahydrofurane, le diméthylformamide ou l'acétonitrile, à une température variant de -78°C à 100°C. Finalement, les dérivés (XI) peuvent être obtenus par une réaction de couplage entre un dérivé (XV) et un composé (XVI) dans lequel D représente un groupe organométallique (par exemple un dérivé du bore, un dérivé de l'étain ou un organozincique) en présence d'une espèce organométallique telle qu'un dérivé du palladium, en présence ou non d'une phosphine telle que la tricyclohexylphosphine ou la triphénylphosphine, en présence d'une base comme le carbonate de potassium ou le fluorure de potassium dans un solvant ou mélange de solvants tel que le dioxane, le diméthylformamide, l'éthylène glycol diméthyléther, le tétrahydrofurane ou l'eau, à une température variant de la température ambiante à 100°C.

Le schéma 5 présente une synthèse alternative des composés de formule (XI).

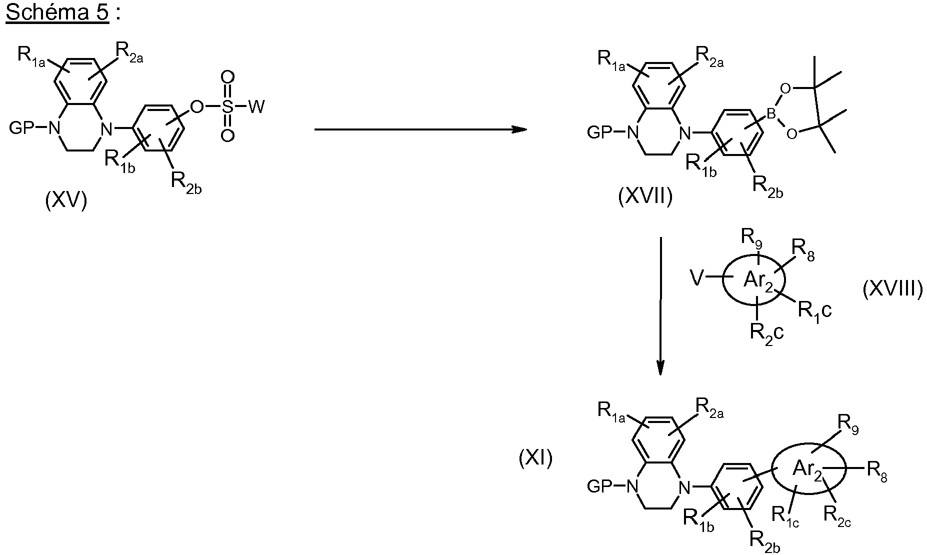

Dans le schéma 5, les composés de formule (XVII) peuvent être préparés par transformation de la fonction ester sulfonique des composés (XV) en une fonction ester boronique pour obtenir les composés (XVII) par une réaction avec le bispinacolatodiborane en présence d'un complexe du palladium tel que le 1,1'-bis(diphénylphosphino)ferrocedichloropalladium (II) en présence d'une base comme l'acétate de potassium et de chlorure de lithium dans un solvant ou mélange de solvants tel que le dichlorométhane, le dioxane ou le diméthylsulfoxyde, à une température variant de la température ambiante à 100°C. Dans une deuxieme étape, les dérivés (XI) peuvent être obtenus par une réaction de couplage entre le dérivé (XVII) et un composé (XVIII) présentant un groupe partant V (par exemple un halogène, un triflate, un nonaflate) en présence d'un catalyseur organométallique tel qu'un dérivé du palladium, en présence ou non d'une phosphine telle que la tricyclohexylphosphine ou la triphénylphosphine, en présence d'une base comme le carbonate de sodium ou potassium ou le fluorure de potassium, dans un solvant ou mélange de solvants tel que le dioxane, le diméthylformamide, l'éthylène glycol diméthyléther, le tétrahydrofurane ou l'eau, à une température variant de la température ambiante à 100°C.

Le schéma 6 détaille une synthèse des composés de formule (VIII) dans lesquels Ar₁ représente un noyau pyridine (un seul des deux atomes Y est un azote, l'autre est un carbone) et A représente une liaison ; ces composés seront appelés ci-après composés de formule (XIX).

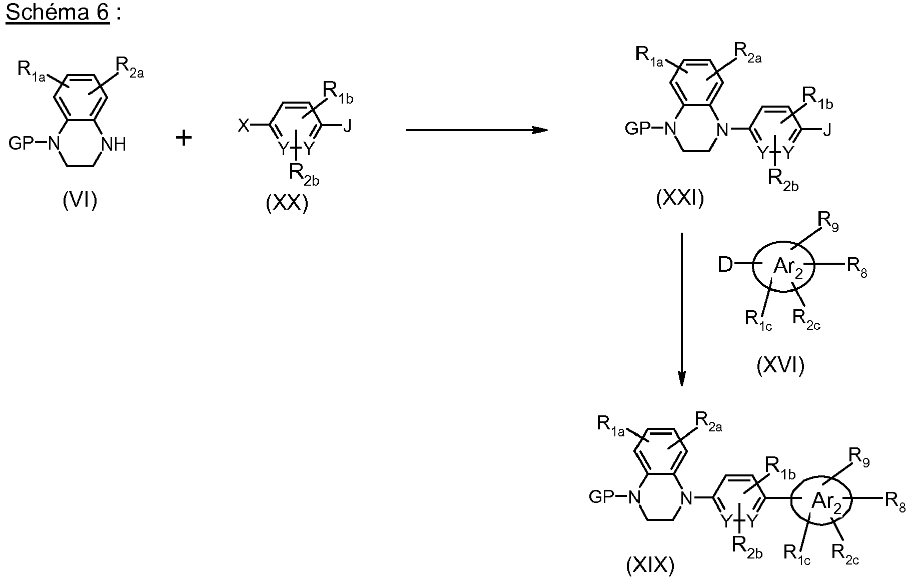

Dans le schéma 6, les composés de formule (XXI) peuvent être préparés par une réaction de substitution nucléophile aliphatique ou aromatique entre une tétrahydroquinoxaline monoprotégée de formule (VI) et un dérivé (XX) présentant un groupe partant X (par exemple un atome de fluor) et un groupe partant J (par exemple un atome de brome) en présence d'une base comme le tertbutylate de potassium ou l'hydrure de sodium dans un solvant tel que la N-méthyle pyrrolidinone ou le diméthylformamide, à une température variant de la température ambiante à 100°C. Finalement, les dérivés (XIX) peuvent être obtenus par une réaction de couplage entre un dérivé (XXI) et un composé (XVI) dans lequel D est soit un groupe organométallique (par exemple un dérivé du bore, un dérivé de l'étain ou un organozincique) soit un atome d'hydrogène quand il est directement connecté à l'atome d'azote d'une amine d'un hétérocycloalkyle, en présence d'un catalyseur organométallique tel qu'un dérivé du palladium, en présence ou non d'une phosphine telle que la tricyclohexylphosphine ou la triphénylphosphine, en présence d'une base comme le carbonate de potasium ou cesium, le triphosphate de potassium, le tert-butylate de sodium ou potassium, ou le fluorure de potassium, dans un solvant ou mélange de solvants tel que le toluène, le dioxane, le diméthylformamide, l'éthylène glycol diméthyléther, le tétrahydrofurane ou l'eau, à une température variant de la température ambiante à 100°C.

Le schéma 7 détaille une synthèse des composés de formule (VIII) dans lesquels Ar₂ est un groupe pipérazine, A est une liaison simple connectée directement sur un des deux atomes d'azote de la pipérazine, R_{1c} est connecté sur l'autre atome d'azote de la pipérazine; ces composés seront appelés ci-après composés de formule (XXII).

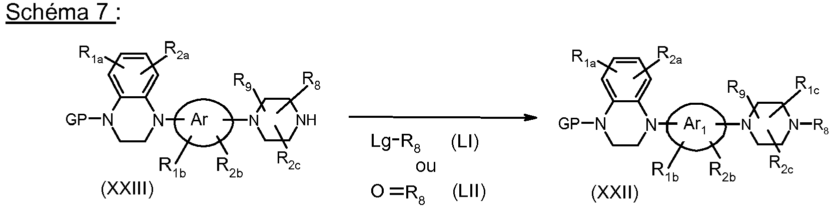

Les composés (XXII) peuvent être obtenus suivant différentes réactions :
- On peut faire réagir sur le composé (XXIII), un dérivé (LI) de type chlorure de sulfonyle, chlorure d'acide ou chlorure de carbamoyle (Lg est alors un atome de chlore) en présence d'une base telle que la triéthylamine, la diisopropyléthylamine ou la pyridine, avec ou sans solvant tel que le dichlorométhane, le chloroforme, le tétrahydrofurane ou le dioxane à une température variant de 0 à 40°C.
- Une réaction d'alkylation est aussi possible entre le composé (XXIII) et un dérivé (LI) dans lequel Lg est par exemple un atome de chlore, de brome ou d'iode, un groupe tosylate ou triflate, en présence d'une base telle que la triéthylamine, la diisopropyléthylamine, dans un solvant tel que le tétrahydrofurane ou le dioxane à une température variant de 0 à 80°C.
- Une réaction d'amination réductrice peut aussi être réalisée entre le composé (XXIII) et un dérivé (LII) de type aldéhyde ou cétone, en utilisant un réducteur tel que le borohydrure de sodium, le triacétoxyborohydrure de sodium ou le cyanoborohydrure de sodium, en présence ou non d'un acide de Bronsted (tel que l'acide chlorhydrique) ou de Lewis (tel que le tétraisopropoxyde de titane) dans un solvant tel que le dichloroéthane, le dichlorométhane, l'acide acétique ou le méthanol, à des températures comprises entre -10°C et 30°C.

Le schéma 8 détaille une synthèse des composés de formule (I) dans lesquels Ar₂ est un groupe pipéridine, A est une liaison simple connectée directement sur l'azote de la pipéridine, R₈ est un hétérocycloalkyle qui est connecté en position 4 de l'atome d'azote de la pipéridine; ces composés seront appelés ci-après composés de formule (XXXVI).

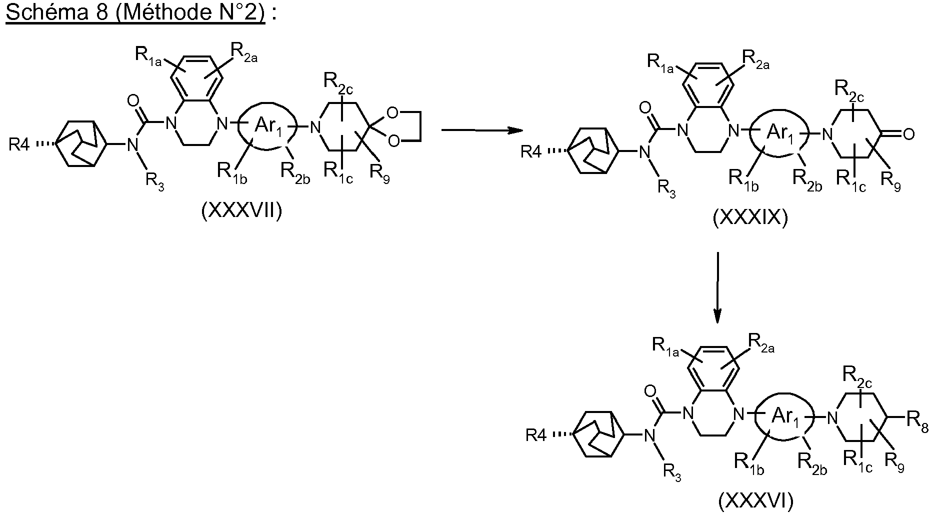

Dans le schéma 8, les dérivés (XXXIX) sont obtenus par hydrolyse de la fonction acétal cyclique du composé (XXXVII) au moyen d'un acide tel que l'acide chlorhydrique dans un solvant ou mélange de solvants tel que l'eau, un alcool, du dioxane à une température variant de la température ambiante à 100°C pour conduire aux cétones (XXXIX). La dernière étape consiste en une réaction d'amination réductrice qui peut être réalisée entre le composé (XIX) et un hétérocycle présentant une fonction amine, en utilisant un réducteur tel que le borohydrure de sodium, le triacétoxyborohydrure de sodium ou le cyanoborohydrure de sodium, en présence ou non d'un acide de Bronsted (tel que l'acide chlorhydrique) ou de Lewis (tel que le tétraisopropoxyde de titane) dans un solvant tel que le dichloroéthane, le dichlorométhane, l'acide acétique ou le méthanol, à des températures comprises entre -10°C et 30°C

Le schéma 9 détaille une synthèse des composés de formule (XVI) dans lesquels Ar₂ est un groupe piperidine, R₈ et R₉ sont connectés en position 4 de l'atome d'azote de la pipéridine et forme un groupe un spiro halogénocyclopropyle ; ces composés seront appelés ci-après composés de formule (XXXX).

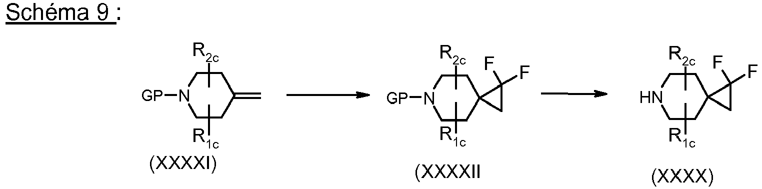

Dans le schéma 9, les dérivés difluorocyclopropanes (XXXXII) sont obtenus par cyclopropanation de la double liaison des composés (XXXXI) au moyen de trimethylsilyl 2-(fluorosulfonyl)difluoroacétate en présence d'une source d'ions fluorure tel que NaF éventuellement dans un solvant tel que le xylène à une température variant de la température ambiante à 150°C. Les amines (XXXX) sont obtenues par déprotection de la fonction amine des composés de formule (XXXXII), par des méthodes choisies parmi celles connues de l'homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Le schéma 10 détaille une synthèse des composés de formule (XVI) dans lesquels Ar₂ est un groupe piperidine, R₈ est connecté en position 4 de l'atome d'azote de la pipéridine et représente une fonction alcoxy-imino ; ces composés seront appelés ci-après composés de formule (XXIII).

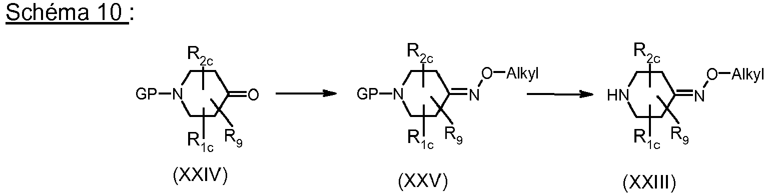

Dans le schéma 10, les oximes (XXV) sont obtenues par la transformation de la cétone des composés (XXIV) au moyen de O-alkylhydroxyamine sous forme de base ou de chlorhydrate en présence ou absence d'une base telle que l'acétate de sodium ou la triéthylamine dans un solvant tel que le méthanol ou l'éthanol à une température variant 0°C à la température ambiante. Les amines (XXIII) sont obtenues par déprotection de la fonction amine des composés de formule (XXV), par des méthodes choisies parmi celles connues de l'homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Le schéma 11 détaille une synthèse des composés de formule (VIII) dans lesquels Ar₂ représente un noyau piperidine lié à Ar₁ par l'atome d'azote et dans lesquels R₈ et R₉ sont connectés en position 4 de l'atome d'azote de la pipéridine et forme ensemble un groupe spiro cyclopropyle substitué par une fonction alkyle ester et A représente une liaison ; ces composés seront appelés ci-après composés de formule (XXVI).

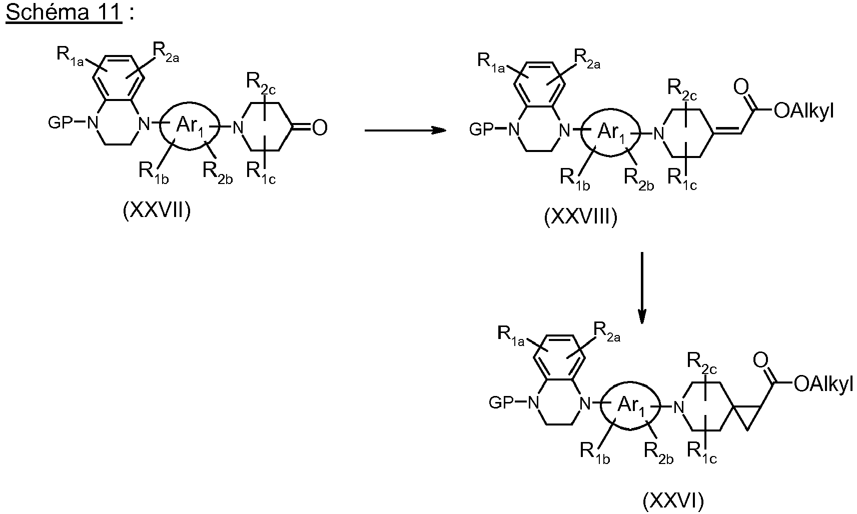

Dans le schéma 11, les dérivés (XXVIII) sont obtenus par la transformation de la cétone des composés (XXVII) au moyen d'une réaction de Wittig-Horner en utilisant par exemple un ylure de phosphonate tel que le diéthyl((éthoxycarbonyl)méthyl)phosphonate en présence d'une base telle que NaH ou tBuOK dans un solvant tel que le THF ou le DMSO à une température variant de 0°C à la température ambiante. Finalement, les composés (XXVI) sont obtenus par cyclopropanation des dérivés éthyléniques (XXVIII) au moyen d'une réaction de cyclopranation de type Corey-Chaykovsky en utilisant par exemple l'iodure de triméthylsulfoxonium en présence d'une base telle que le NaH ou le tBuOK dans un solvant tel que le DMSO.

Le schéma 12 détaille une synthèse des composés de formule (XVI) dans lesquels Ar₂ est un groupe [1,4]azasilinane, R_{1c} et R_{2c} sont des atomes d'hydrogène, R₈ et R₉ sont connectés sur l'atome de silicium ; ces composés seront appelés ci-après composés de formule (XXIX).

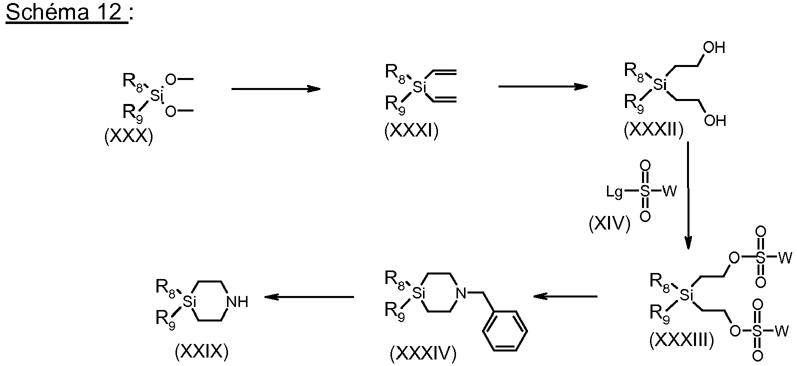

Dans le schéma 12, les dérivés (XXXI) sont obtenus par addition d'un dérivé organométallique tel que le vinylmagnésien sur les composés (XXX) dans un solvant tel que le THF ou l'éther à une température variant de la température ambiante à 80°C. La réaction suivante est une hydroboration des doubles liaisons des dérivés (XXXI) pour former les alcools (XXXII) au moyen d'un dérivé du bore tel que le 9-BBN ou BH₃, puis oxydation en milieu alcalin par exemple avec un mélange de H₂O₂ et de soude. On transforme ensuite les deux fonctions hydroxyle des composés (XXXII) en fonction ester sulfonique pour former les composés (XXXIII) par action d'un dérivé sulfonique (XIV) où SO₂W représente par exemple un groupe mésylate, tosylate, triflate ou nanoflate, tel qu'un anhydride sulfonique (Lg=OSO₂W), un fluorure d'acide sulfonique (Lg=F) ou un chlorure d'acide sulfonique (Lg=Cl), en présence d'une base ou d'un mélange de bases comme la triéthylamine, la pyridine, la diméthylaminopyridine, la diisopropyléthylamine ou le carbonate de potassium dans un solvant ou mélange de solvants tel que le dichlorométhane, le chloroforme, le toluène, le tétrahydrofurane, le diméthylformamide ou l'acétonitrile, à une température variant de -78°C à 100°C. La réaction suivante qui peut être réalisée en même temps que l'étape précédente est la substitution nucléophile des deux fonctions ester sulfonique des composés (XXXIII) par la benzylamine pour conduire aux hétérocycles (XXXIV). Les dérivés (XXIX) sont finalement obtenus par élimination du groupe benzyle porté par le groupe amino des composés (XXXIV). Les méthodes de déprotection possibles comprennent entre autres l'utilisation d'hydrogène en présence d'un catalyseur dérivé du palladium pour réaliser une réaction d'hydrogénolyse, dans un solvant ou mélange de solvants tel que le méthanol, l'éthanol, l'acétate d'éthyle, le tétrahydrofurane, sous une pression d'hydrogène comprise entre 1 et 10 bars à une température variant de la température ambiante à 80°C. Une méthode alternative pour réaliser l'élimination du groupe benzyle sur une amine secondaire consiste à mettre en oeuvre la réaction d'Olofson (telle que décrite dans Tett. Lett. 1977, page 1570, et J.Org. Chem. 1990, 55, page 1) dans laquelle on utilise un chloroformiate tel que le chloroformiate de vinyle ou de chloroéthyle qui permet de conduire aux hétérocycles (XXIX) sous forme de chlorhydrate après traitement ou non avec une solution aqueuse d'HCl.

Dans les schémas qui précèdent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

L'invention, selon un autre de ses aspects, a également pour objet les composés de formules (II), (IV), (VIII), (X), (XI), (XIII), (XV), (XVI), (XVII), (XXI), (XIX), (XXII), (XXVIII), (XXXVII), (XXXVI), etc.. définies ci-avant. Ces composés sont utiles comme intermédiaires de synthèse des composés de formule (I).

Les abréviations et formules brutes suivantes sont utilisées :
- 9-BBN: 9-Borabicyclo[3.3.1]nonane
- °C: Degré Celsius
- DME: diméthoxyéthane
- DMF: diméthylformamide
- DMSO: diméthyl sulfoxyde
- h: heure(s)
- H₂: dihydrogène
- H₂O: eau
- HCl: acide chlorhydrique
- K₂CO₃: Carbonate de potassium
- LC/MS: chromatographie liquide/ spectrométrie de masse
- ml ou mL: millilitre(s)
- mmol: millimole(s)
- MHz: MégaHertz
- MgSO₄: Sulfate de magnésium
- N: normal
- NMP: N-méthylmorpholine
- NaHCO₃: Hydrogénocarbonate de sodium
- Pd/C: Palladium sur charbon
- P₂O₅: Pentoxyde de phosphore
- ppm: parties par millions
- psi: pound per square inch
- SO₂: dioxyde de soufre

### Exemple 1 : Trans (5-hydroxy-adamantan-2-yl)-amide de l'acide 4-(4-tert-butoxyimino-3,4,5,6-tétrahydro-2H-[1,3']bipyridinyl-6'-yl)-3,4-dihydro-2H-quinoxaline-1-carboxylique (composé n°2)

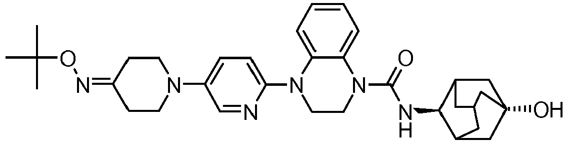

### 1.1 : Ester tert-butylique de l'acide 4-(5-bromo-pyridin-2-yl)-3,4-dihydro-2H-quinoxaline-1-carboxylique

On place 30 g d'ester *tert*-butylique de l'acide 3,4-dihydro-2H-quinoxaline-1-carboxylique dans 430 ml de *N*-méthyl pyrrolidinone à 0°C sous azote. On ajoute par portion 30 g de *tert*-butylate de potassium en maintenant la température en dessous de 10°C. On agite 1h30 à température ambiante, puis à 0°C on ajoute 850 ml d'eau et 800 ml d'éther éthylique. La phase aqueuse est extraite avec 800 ml d'éther éthylique, puis avec 400 ml d'éther éthylique. Les phases organiques sont rassemblées puis séchées sur sulfate de magnésium et concentrée à sec. On ajoute ensuite 300 ml de pentane au brut réactionnel et on sonique le mélange hétérogène obtenu sous ultra-sons pendant 5 min. Le mélange est ensuite placé pendant 48h à 5°C, puis le solide est filtré, lavé trois fois au pentane puis séché à 40°C pendant 5h. On obtient 35 g d'ester *tert*-butylique de l'acide 4-(5-bromo-pyridin-2-yl)-3,4-dihydro-2H-quinoxaline-1-carboxylique.
M+H⁺=392.0

### 1.2 : Ester tert-butylique de l'acide 4-tert-butoxyimino-pipéridine-1-carboxylique

On place 0,662 g de chlorhydrate de O-*tert*-butylhydroxylamine dans un ballon de taille approprié et on ajoute 12 ml d'éthanol, puis 0,432 g d'acétate de sodium. Le mélange réactionnel est ensuite porté à reflux pendant 15 minutes, puis on additionne 1 g d'ester *tert*-butylique de l'acide 4-oxo-pipéridine-1-carboxylique préalablement solubilisé dans 13 ml d'éthanol. Après 1h30 de chauffage, le milieu réactionnel est ensuite refroidi à température ambiante et l'éthanol est évaporé sous pression réduite. Le résidu est repris par du dichlorométhane, séché sur sulfate de sodium, filtré, concentré à sec et séché sous vide. On obtient 1,32 g d'ester *tert*-butylique de l'acide 4-*tert*-butoxyimino-pipéridine-1-carboxylique (97%) sous forme d'un solide blanc.
(M+H⁺)=271

### 1.3 : Pipéridin-4-one O-tert-butyl-oxime

Une solution de 34 ml de dichlorométhane contenant 0,9 g d'ester *tert*-butylique de l'acide 4-*tert*-butoxyimino-pipéridine-1-carboxylique est refroidie à 0°C. 25ml d'une solution d'acide chlorhydrique 4M dans le dioxane (30 éq) sont additionnés. Le mélange réactionnel est agité à température ambiante pendant 3 heures puis dilué par ajout de dichlorométhane. Une solution saturée d'hydrogénocarbonate de sodium est ensuite ajoutée jusqu'à obtention d'un pH=8. La phase aqueuse est extraite trois fois avec du dichlorométhane. Les phases organiques sont ensuite regroupées, séchées sur sulfate de sodium, filtrées et concentrées à sec sous pression réduite. On obtient 0,49 g de pipéridin-4-one O-*tert*-butyl-oxime.
(M+H⁺)=171

### 1.4: Ester tert-butylique de l'acide 4-(4-tert-butoxyimino-3,4,5,6-tétrahydro-2H-[1,3']bipyridinyl-6'-yl)-3,4-dihydro-2H-quinoxaline-1-carboxylique

A une solution de 8 mL de toluène anhydre contenant 0,5 g d'ester *tert*-butylique de l'acide 4-(5-bromo-pyridin-2-yl)-3,4-dihydro-2H-quinoxaline-1-carboxylique (intermédiaire 1.1) sont ajoutés 0,229 g de pipéridin-4-one O-*tert*-butyl-oxime, 0,172 g de *tert*-butylate de sodium, 0,084 g de dicyclohexyl-(2',6'-diméthoxy-biphényl-2-yl)-phosphane et 0,047 g de tris(dibenzylidèneacetone)dipalladium (0). Le milieu réactionnel est chauffé à 115°C. Après 2 heures de chauffage, la solution est refroidie à température ambiante puis filtrée sur célite. Les solvants sont évaporés sous pression réduite. Le résidu est repris par de l'acétate d'éthyle. La phase organique est ensuite lavée à l'eau, séchée sur sulfate de sodium, filtrée et concentrée à sec sous pression réduite. Le résidu obtenu est purifié par chromatographie sur colonne de silice en éluant avec un gradient d'un mélange de dichlorométhane/méthanol (95/5 à 60/40) et 0,526 g d'ester tert-butylique de l'acide 4-(4-*tert*-butoxyimino-3,4,5,6-tétrahydro-2H-[1,3']bipyridinyl-6'-yl)-3,4-dihydro-2H-quinoxaline-1-carboxylique sont obtenus.
(M+H⁺)=480

### 1.5. 6'-(3,4-Dihydro-2H-quinoxalin-1-yl)-2,3,5,6-tétrahydro-[1,3']bipyridinyl-4-one O-tert-butyl-oxime

Une solution de 11 mL de dichlorométhane contenant 0,526 g de ester *tert*-butylique de l'acide 4-(4-*tert*-butoxyimino-3,4,5,6-tétrahydro-2H-[1,3']bipyridinyl-6'-yl)-3,4-dihydro-2H-quinoxaline-1-carboxylique est refroidie à 0°C. 3,6 ml d'une solution d'acide chlorhydrique 4M dans le dioxane sont additionnés. Le milieu réactionnel est agité à froid pendant 3 heures puis est dilué avec du dichlorométhane. On ajoute ensuite de l'eau puis du carbonate de sodium jusqu'à obtention d'un pH=12. La phase aqueuse est extraite trois fois avec du dichlorométhane. Les phases organiques sont ensuite regroupées, séchées sur sulfate de sodium, filtrées et concentrées à sec sous pression réduite. On obtient 0,485 g de 6'-(3,4-dihydro-2H-quinoxalin-1-yl)-2,3,5,6-tétrahydro-[1,3']bipyridinyl-4-one O-*tert*-butyl-oxime.
(M+H⁺)=380

### 1.6 : trans-(5-Hydroxy-adamantan-2-yl)-amide de l'acide 4-(4-tert-butoxyimino-3,4,5,6-tétrahydro-2H-[1,3']bipyridinyl-6'-yl)-3,4-dihydro-2H-quinoxaline-1-carboxylique

Une solution sous azote de 13 ml de dichlorométhane anhydre contenant 0,485 g de 6'-(3,4-dihydro-2H-quinoxalin-1-yl)-2,3,5,6-tétrahydro-[1,3']bipyridinyl-4-one O-*tert-*butyl-oxime et 0,35 mL de triéthylamine est refroidie à 0°C. 0,151 g de triphosgène sont ensuite ajoutés. Après 2 heures d'agitation à température ambiante, 1 g de l'alcool *trans*-4-amino-adamantan-1-ol, 0,36 ml de triéthylamine (2 éq) et 1 ml de diméthylformamide anhydre sont additionnés. L'agitation est maintenue pendant 18 heures. Les solvants sont évaporés sous pression réduite et le résidu est repris par de l'eau, puis une solution de carbonate de sodium est additionnée jusqu'à obtention d'un pH basique. La phase aqueuse est extraite trois fois avec du dichlorométhane. Les phases organiques sont ensuite regroupées, séchées sur sulfate de sodium, filtrées et concentrées à sec sous pression réduite. Le résidu obtenu est purifié par chromatographie sur colonne de silice en éluant avec un gradient d'un mélange dichlorométhane/méthanol (95/5 à 0/100). On obtient 0,428 g de *trans*-(5-hydroxy-adamantan-2-yl)-amide de l'acide 4-(4-*tert*-butoxyimino-3,4,5,6-tétrahydro-2H-[1,3']bipyridinyl-6'-yl)-3,4-dihydro-2H-quinoxaline-1-carboxylique.
(M+H⁺)=573, PF=110-113°C ;
1 H NMR (400 MHz, DMSO-d6) δ(ppm) = 8,07 (d, J = 3 Hz, 1 H), 7.44 (dd, J = 7,9 Hz et 1,5 Hz, 1 H), 7,40 (dd, J = 9 Hz et 3 Hz, 1 H), 7,11 (m, 2H), 6,94 (m, 1 H), 6,86 (m, 1 H), 6,00 (d, J = 6 Hz, 1 H), 4,38 (s, 1 H), 3,79 (m, 4H), 3,70 (m, 1 H), 3,33 (m, 2H), 3,27 (m, 2H), 2,61 (m, 2H), 2,41 (m, 2H), 2,04 (m, 2H), 1.99 (m, 1H), 1,73 à 1,57 (m, 8H), 1,36 (m, 2H), 1,25 (s, 9H).

### Exemple 2 : trans-(5-Carbamoyl-adamantan-2-yl)-amide de l'acide 4-{5-[4-(2,2-difluoro-cyclopropylméthyl)-pipérazin-1-yl]-pyridin-2-yl}-3,4-dihydro-2H-quinoxaline-1-carboxylique (composé n°4)

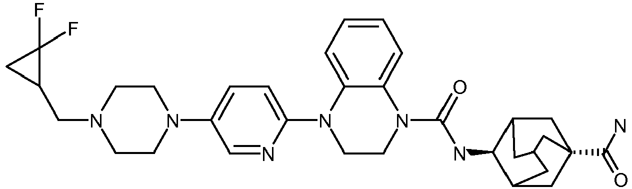

### 2.1 : Ester tert-butylique de l'acide 4-[5-(4-benzyloxycarbonyl-pipérazin-1-yl)-pyridin-2-yl]-3,4-dihydro-2H-quinoxaline-1-carboxylique

On mélange 10,12 g d'ester *tert*-butylique de l'acide 4-(5-bromo-pyridin-2-yl)-3,4-dihydro-2H-quinoxaline-1-carboxylique (intermédiaire 1.1) et 5,7 g de 4-carboxybenzyle pipérazine dans 118 ml de toluène, puis on ajoute 0,95 g de tris(dibenzylidèneacétone)dipalladium (0), 1,7 g de 2-dicyclohexylphosphino-2',6'-diméthoxybiphényl et 3,5 g de *tert*-butylate de sodium. Le milieu réactionnel est chauffé à 110°C pendant 3 h. On ajoute ensuite de l'acétate d'éthyle et le mélange est lavé une fois à l'eau et une fois avec une solution aqueuse saturée de chlorure de sodium. La phase organique est séchée sur sulfate de magnésium et concentrée sous pression réduite. Le brut obtenu est chromatographié sur gel de silice en éluant avec un gradient d'un mélange d'heptane/acétate d'éthyle (90/10 à 0/100). On obtient 10,16 g d'ester *tert*-butylique de l'acide 4-[5-(4-benzyloxycarbonyl-pipérazin-1-yl)-pyridin-2-yl]-3,4-dihydro-2H-quinoxaline-1-carboxylique.
M+H⁺= 530,5

### 2.2 : Ester tert-butylique de l'acide 4-(5-pipérazin-1-yl-pyridin-2-yl)-3,4-dihydro-2H-quinoxaline-1-carboxylique

Dans un flacon de parr, on place 5,08 g de l'ester *tert*-butylique de l'acide 4-[5-(4-benzyloxycarbonyl-pipérazin-1-yl)-pyridin-2-yl]-3,4-dihydro-2H-quinoxaline-1-carboxylique dans 240 ml d'éthanol puis on ajoute 2 g de Pd/C 10% (50% dans l'eau). On agite le milieu réactionnel ainsi obtenu à 35°C sous 45 psi d'hydrogène pendant 3,5h. On filtre ensuite sous atmosphère inerte sur filtre Whatman puis on rince de nombreuses fois au méthanol qui est ensuite évaporé sous pression réduite. On obtient 3,57 g d'ester *tert*-butylique de l'acide 4-(5-pipérazin-1-yl-pyridin-2-yl)-3,4-dihydro-2H-quinoxaline-1-carboxylique.
M+H⁺= 396,6

### 2.3: Ester tert-butylique de l'acide 4-{5-[4-(2,2-difluoro-cyclopropylméthyl)-pipérazin-1-yl]-pyridin-2-yl}-3,4-dihydro-2H-quinoxaline-1-carboxylique

On ajoute 0,3 g d'ester *tert*-butylique de l'acide 4-(5-pipérazin-1-yl-pyridin-2-yl)-3,4-dihydro-2H-quinoxaline-1-carboxylique dans 10 mL d'acétonitrile, auquel on ajoute 0,189 g de K₂CO₃ puis I0,143 g de 2-bromométhyl-1,1-difluoro-cyclopropane. Le milieu réactionnel est agité 2h à température ambiante sous azote, puis 18h au reflux sous azote. Le milieu réactionnel est ensuite refroidi à température ambiante, versé dans 100 mL d'H₂O et extrait 3 fois avec 50 ml d'EtOAc. Les phases organiques résultantes sont combinées, lavées avec 100 ml d'H₂O, 100 ml d'une solution aqueuse saturée en chlorure de sodium, séchées sur MgSO₄, filtrées et concentrées à sec. Le produit brut obtenu est chromatographié sur gel de silice en éluant avec un gradient de méthanol dans le dichlorométhane variant de 1% à 8%. On obtient 0,3 g d'ester *tert*-butylique de l'acide 4-{5-[4-(2,2-difluoro-cyclopropylméthyl)-pipérazin-1-yl]-pyridin-2-yl}-3,4-dihydro-2H-quinoxaline-1-carboxylique.
[M+H⁺] = 486

### 2.4: 1-{5-[4-(2,2-Difluoro-cyclopropylméthyl)-pipérazin-1-yl]-pyridin-2-yl}-1,2,3,4-tétrahydro-quinoxaline

On place 0,3 g d'ester *tert*-butylique de l'acide 4-{5-[4-(2,2-difluoro-cyclopropylméthyl)-pipérazin-1-yl]-pyridin-2-yl}-3,4-dihydro-2H-quinoxaline-1-carboxylique dans 5 mL de dioxane, auquel on ajoute 2,32 ml d'HCl 4N dans le dioxane. Le milieu réactionnel est agité à température ambiante en milieu clos pendant 18h. On ajoute à nouveau 2,32 mL d'HCl 4N dans le dioxane et le milieu réactionnel est agité 18h supplémentaires à température ambiante. On ajoute à nouveau 2,32 ml d'HCl 4N dans le dioxane et le milieu réactionnel est agité 18h supplémentaires à température ambiante. Après concentration à sec, le milieu réactionnel est dilué par une solution de 50 ml d'HCl 1 N dans l'eau et extrait avec 100 ml d'éther diéthylique. La phase organique est relavée avec 50 ml d'HCl 1N dans l'eau. Les phases aqueuses sont ensuite combinées, basifiées avec K₂CO₃ en poudre jusqu'à pH 10, extraites 3 fois avec 50 ml de dichlorométhane. Les phases organiques résultantes sont combinées, lavées avec une solution aqueuse saturée en chlorure de sodium, séchées sur MgSO₄ et concentrées à sec. On obtient 0,24 g de 1-{5-[4-(2,2-difluoro-cyclopropylméthyl)-pipérazin-1-yl]-pyridin-2-yl}-1,2,3,4-tétrahydro-quinoxaline.
[M+H⁺] = 386

### 2.5 : trans-(5-Carbamoyl-adamantan-2-yl)-amide de l'acide 4-{5-[4-(2,2-difluoro-cyclopropylméthyl)-pipérazin-1-yl]-pyridin-2-yl}-3,4-dihydro-2H-quinoxaline-1-carboxylique

On place 0,24 g de 1-{5-[4-(2,2-difluoro-cyclopropylméthyl)-pipérazin-1-yl]-pyridin-2-yl}-1,2,3,4-tétrahydro-quinoxaline dans 5 mL de dichlorométhane à 0°C. On ajoute 0,17 mL de triéthylamine puis 0,073 g de triphosgène. Le milieu réactionnel est agité pendant 30 min sous azote à 0°C, puis 3 heures à température ambiante. On ajoute alors 0,16 g de chlorhydrate de l'amide de l'acide *trans*-4-amino-adamantane-1-carboxylique, 0,22 mL de triéthylamine et 5mL de DMF. Le milieu réactionnel est agité 18h à température ambiante sous azote. Après hydrolyse avec 100ml d'H₂O, le milieu est extrait 2 fois avec 50ml de dichlorométhane. Les phases organiques sont combinées, lavées 2 fois avec 100ml d'H₂O puis avec 100ml d'une solution aqueuse saturée en chlorure de sodium, séchées sur MgSO₄ et concentrées à sec. Le brut obtenu est chromatographié sur gel de silice en éluant avec un gradient de méthanol dans du dichlorométhane variant de 1% à 10%. Après trituration dans l'éther diéthylique, filtration et séchage, on obtient 0.25 g de *trans*-(5-Carbamoyl-adamantan-2-yl)-amide de l'acide 4-{5-[4-(2,2-difluoro-cyclopropylméthyl)-pipérazin-1-yl]-pyridin-2-yl}-3,4-dihydro-2H-quinoxaline-1-carboxylique.
[M+H⁺] = 606 ; PF = 206°C ;
1 H NMR (400 MHz, DMSO-d6) δ(ppm) = 8,05 (d, J = 3 Hz, 1 H), 7,45 (dd, J = 7,9 Hz et 1,5 Hz, 1 H), 7,39 (dd, J = 9 Hz et 3 Hz, 1 H), 7.11 (m, 2H), 6,97 (s.large, 1 H), 6,86 (m, 1 H), 6,69 (s.large, 1H), 6,06 (d, J = 6 Hz, 1H), 3,79 (m, 4H), 3,75 (m, 1H), 3,15 (m, 4H), 2,70 à 2,53 (m, 5H), 2,39 (m, 1 H), 2,04 à 1.69 (m, 11 H), 1,62 (m, 1 H), 1,45 (m, 2H), 1,19 (m, 2H).

### Exemple 3 : trans-(5-carbamoyl-adamantan-2-yl)-amide de l'acide 4-[4-(1,1-dioxo-1lambda6-thiomorpholin-4-yl)-3,4,5,6-tétrahydro-2H-[1,3']bipyridinyl-6'-yl]-3,4-dihydro-2H-quinoxaline-1-carboxylique (composé n°5)

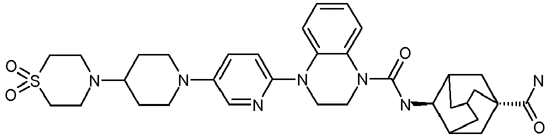

### 3.1 : Ester tert-butylique de l'acide 4-(1,1-dioxo-1lambda6-thiomorpholin-4-yl)-pipéridine-1-carboxylique

On place 1,473 g de boc-4-pipéridone dans 45 mL de méthanol. On ajoute à température ambiante 1 g de thiomorpholine-1,1-dioxide et 0,47 ml d'acide acétique. On observe une prise en masse. On ajoute donc du méthanol jusqu'à agitation correcte. 0,511 g de cyanoborohydrure de sodium sont alors additionnés. On laisse agiter à température ambiante pendant 18h puis la solution est chauffée à reflux pendant 3h. La solution est ensuite évaporée à sec et le produit brut obtenu est chromatographié sur gel de silice en éluant avec un gradient d'acétate d'éthyle dans l'heptane variant de 20 à 100%. On obtient 0,786 g de l'ester *tert*-butylique de l'acide 4-(1,1-dioxo-1lambda6-thiomorpholin-4-yl)-pipéridine-1-carboxylique.
M-56+H⁺=263

### 3.2 : 4-Pipéridin-4-yl-thiomorpholine 1,1-dioxyde

On place 0,786 g de l'ester *tert*-butylique de l'acide 4-(1,1-dioxo-1lambda6-thiomorpholin-4-yl)-pipéridine-1-carboxylique dans 5 ml de dichlorométhane. On ajoute à température ambiante 12,3 ml d'acide chlorhydrique 4M dans le dioxane. La solution est agitée pendant 18h et évaporée à sec. Le produit ainsi obtenu est repassé à l'état de base à l'aide d'une résine tétralkylammonium carbonate à raison de 2 g par mmole. On obtient 0,555 g de 4-pipéridin-4-yl-thiomorpholine 1,1-dioxyde.

### 3.3: Ester tert-butylique de l'acide 4-[4-(1,1-dioxo-1lambda6-thiomorpholin-4-yl)-3,4,5,6-tétrahydro-2H-[1,3']bipyridinyl-6'-yl]-3,4-dihydro-2H-quinoxaline-1-carboxylique.

On place 0,993 g de l'ester *tert*-butylique de l'acide 4-(5-bromo-pyridin-2-yl)-3,4-dihydro-2H-quinoxaline-1-carboxylique (intermédiaire 1.1) dans 12 mL de toluène. On ajoute à température ambiante 0,555 g de 4-pipéridin-4-yl-thiomorpholine 1,1-dioxide, 0,342 g de *tert*-butylate de sodium, 0,167 g de 2-dicyclohexylphosphino-2',6'-diméthoxybiphényl et 0.093 g de tris(dibenzylidèneacétone)dipalladium (0) et le milieu réactionnel est chauffé pendant 3h à 110°C. On ajoute ensuite de l'acétate d'éthyle et le mélange est décanté. On extrait une deuxième fois la phase aqueuse à l'acétate d'éthyle et on lave les phases organiques à l'eau. Elles sont alors séchées sur sulfate de magnésium et concentrées sous pression réduite. Le brut obtenu est chromatographié sur gel de silice en éluant avec un gradient de méthanol dans le dichlorométhane variant de 1% à 10%. On obtient 0,86 g de l'ester *tert*-butylique de l'acide 4-[4-(1,1-dioxo-1lambda6-thiomorpholin-4-yl)-3,4,5,6-tétrahydro-2H-[1,3']bipyridinyl-6'-yl]-3,4-dihydro-2H-quinoxaline-1-carboxylique.
M+H⁺=528

### 3.4 : Chlorhydrate du 1-[4-(1,1-dioxo-1lambda6-thiomorpholin-4-yl)-3,4,5,6-tétrahydro-2H-[1,3']bipyridinyl-6'-yl]-1,2,3,4-tétrahydro-quinoxaline.

On place 0,4 g de l'ester *tert*-butylique de l'acide 4-[4-(1,1-dioxo-1lambda6-thiomorpholin-4-yl)-3,4,5,6-tétrahydro-2H-[1,3']bipyridinyl-6'-yl]-3,4-dihydro-2H-quinoxaline-1-carboxylique.dans 4 ml de dichlorométhane. On ajoute à température ambiante 3,79 mL d'acide chlorhydrique 4M dans le dioxane. La solution est agitée pendant 2 jours. On évapore à sec. On obtient 0,38 g de chlorhydrate du 1-[4-(1,1-dioxo-1lambda6-thiomorpholin-4-yl)-3,4,5,6-tétrahydro-2H-[1,3']bipyridinyl-6'-yl]-1,2,3,4-tétrahydro-quinoxaline.
M+H⁺=428

### 3.5 : trans-(5-Carbamoyl-adamantan-2-yl)-amide 4-[4-(1,1-dioxo-1lambda6-thiomorpholin-4-yl)-3,4,5,6-tétrahydro-2H-[1,3']bipyridinyl-6'-yl]-3,4-dihydro-2H-quinoxaline-1-carboxylique.

Dans un ballon de 100 ml, on place 0,379 g de chlorhydrate du 1-[4-(1,1-dioxo-1lambda6-thiomorpholin-4-yl)-3,4,5,6-tétrahydro-2H-[1,3']bipyridinyl-6'-yl]-1,2,3,4-tétrahydro-quinoxaline dans 13 mL d'une solution saturée d'hydrogénocarbonate de sodium. On ajoute 13 ml de dichlorométhane. La solution est refroidie dans un bain de glace. A +5°C, 0,6 ml d'une solution de phosgène à 20% dans le toluène est ajouté. Au bout de 30 minutes on ajoute de nouveau 0,6 ml de phosgène à 20% dans le toluène puis 30 minutes plus tard, on ajoute encore 0,6 ml de phosgène à 20% dans le toluène. 30 minutes après, on décante le milieu réactionnel. On ajoute du dichlorométhane à la phase aqueuse et on décante à nouveau. Les phases organiques sont alors réunies, séchées sur sulfate de magnésium, filtrées et évaporées à sec. Ce brut réactionnel est repris dans 13 mL de diméthylformamide. On ajoute 0,66 mL de diisopropyléthylamine. On ajoute 0,175 g de l'amide de l'acide *trans* -4-amino-adamantane-1-carboxylique. La solution est ensuite agitée pendant 18h et le milieu réactionnel est versé sur de l'eau et extrait deux fois à l'acétate d'éthyle. La phase organique est lavée deux fois à l'eau, séchée sur sulfate de magnésium, filtrée et évaporée à sec. Le brut obtenu est chromatographié sur gel de silice en éluant avec un gradient de méthanol dans le dichlorométhane variant de 1% à 10%. On obtient 0,115 g de *trans*-(5-Carbamoyl-adamantan-2-yl)-amide 4-[4-(1,1-dioxo-1lambda6-thiomorpholin-4-yl)-3,4,5,6-tétrahydro-2H-[1,3']bipyridinyl-6'-yl]-3,4-dihydro-2H-quinoxaline-1-carboxylique après trituration dans l'éther éthylique avec quelques gouttes d'acétate d'éthyle.
M+H⁺=648, PF=160-200°C ;
1 H NMR (400 MHz, DMSO-d6) δ(ppm) = 8,04 (m, 1 H), 7,42 (m, 2H), 7,10 (m, 2H), 6,95 (m, 2H), 6,86 (m, 1 H), 6,68 (s.large, 1 H), 6,05 (d, J = 6 Hz, 1 H), 3,87 à 3,63 (m, 7H), 3,04 (m, 8H), 2,68 (m, 3H), 2,12 à 1,34 (m, 17H).

### Exemple 4 : Chlorhydrate du trans (5-carbamoyl-adamantan-2-yl)-amide de l'acide 4-[5-(1,1-difluoro-6-aza-spiro[2.5]oct-6-yl)-pyridin-2-yl]-3,4-dihydro-2H-quinoxaline-1-carboxylique (composé n°11)

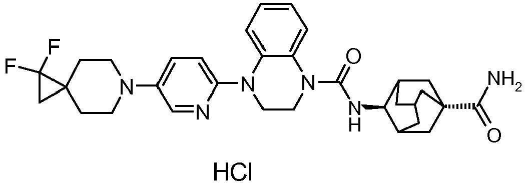

### 4.1 : Ester tert-butylique de l'acide 1,1-difluoro-6-aza-spiro[2.5]octane-6-carboxylique

Dans un ballon de 50 ml, on place 5,07 g de triméthylsilyl-2,2-difluoro-2-(fluorosulfonyl)acétate et 0 ,025 g de fluorure de sodium. Le mélange réactionnel est refroidi à l'aide d'un bain de glace et on ajoute 2 g de 1-*N*-boc-4-méthylènepipéridine goutte à goutte. Le mélange réactionnel est ensuite porté à 105°C. Dès que cette température est atteinte, un fort dégagement gazeux est observé et la solution devient orange très foncé. Le chauffage est alors arrêté et, une fois refroidi, le mélange réactionnel est versé sur une solution d'hydrogénocarbonate de sodium. On extrait deux fois au dichlorométhane et on lave la phase organique deux fois à l'eau. On sèche sur du sulfate de magnésium, on filtre et on concentre à sec. Le brut obtenu est chromatographié sur gel de silice en éluant avec un gradient d'acétate d'éthyle dans l'heptane variant de 0 à 30%. On obtient 1,2 g de l'ester tert-butylique de l'acide 1,1-difluoro-6-aza-spiro[2.5]octane-6-carboxylique.
M-56+ACN+H⁺=233

### 4.2 : Chlorhydrate du 1,1-difluoro-6-aza-spiro[2.5]octane

On place 1,2 g de l'ester *tert*-butylique de l'acide 1,1-Difluoro-6-aza-spiro[2.5]octane-6-carboxylique dans 24 mL de dichlorométhane. On ajoute à température ambiante 24 mL d'acide chlorhydrique 4M dans le dioxane. La solution est agitée pendant 4h et est évaporée à sec. On obtient 1 g de chlorhydrate de 1,1-difluoro-6-aza-spiro[2.5]octane.

### 4.3 : Ester tert-butylique de l'acide 4-[5-(1,1-difluoro-6-aza-spiro[2.5]oct-6-yl)-pyridin-2-yl]-3,4-dihydro-2H-quinoxaline-1-carboxylique

On place 0,894 g de chlorhydrate de 1,1-difluoro-6-aza-spiro[2.5]octane dans 22 ml de toluène. On ajoute à température ambiante 1,12 g de terbutylate de sodium, 1,9 g de l'ester *tert*-butylique de l'acide 4-(5-bromo-pyridin-2-yl)-3,4-dihydro-2H-quinoxaline-1-carboxylique (intermédiaire 1.1), 0,32 g de 2-dicyclohexylphosphino-2',6'-diméthoxybiphényl et 0,178 g de tris(dibenzylidèneacétone)dipalladium (0). On chauffe 3h à 105°C. On ajoute ensuite de l'acétate d'éthyle et le mélange est décanté. On extrait encore deux fois à l'acétate d'éthyle et on lave les phases organiques deux fois à l'eau. Elles sont alors séchées sur sulfate de magnésium et concentrées sous pression réduite. Le brut obtenu est chromatographié sur gel de silice en éluant avec un gradient d'acétate d'éthyle dans l'heptane variant de 10% à 70%. On obtient 1,48 g de l'ester *tert*-butylique de l'acide 4-[5-(1,1-difluoro-6-aza-spiro[2.5]oct-6-yl)-pyridin-2-yl]-3,4-dihydro-2H-quinoxaline-1-carboxylique.
M+H⁺=457

### 4.4 1-[5-(1,1-Difluoro-6-aza-spiro[2.5]oct-6-yl)-pyridin-2-yl]-1,2,3,4-tétrahydro-quinoxaline

On place 1,48 g de l'ester *tert*-butylique de l'acide 4-[5-(1,1-difluoro-6-aza-spiro[2.5]oct-6-yl)-pyridin-2-yl]-3,4-dihydro-2H-quinoxaline-1-carboxylique dans 16 mL de dichlorométhane. On ajoute à +4°C 24 mL d'acide chlorhydrique 4M dans le dioxane. La solution est agitée à température ambiante pendant 4h et évaporée à sec. Le résidu est repris par une solution saturée d'hydrogénocarbonate de sodium jusqu'à pH basique. La solution est extraite deux fois au dichlorométhane, lavée à l'eau, séchée sur sulfate de magnésium, filtrée et évaporée à sec. On obtient 1,4 g de 1-[5-(1,1-difluoro-6-aza-spiro[2.5]oct-6-yl)-pyridin-2-yl]-1,2,3,4-tétrahydro-quinoxaline.
M+H⁺=357

### 4.5 Chlorhydrate du trans-(5-carbamoyl-adamantan-2-yl)-amide de l'acide 4-[5-(1,1-difluoro-6-aza-spiro[2.5]oct-6-yl)-pyridin-2-yl]-3,4-dihydro-2H-quinoxaline-1-carboxylique

Dans un tricol de 50 ml sous atmosphère inerte d'azote, on introduit 1,17 g de 1-[5-(1,1-difluoro-6-aza-spiro[2.5]oct-6-yl)-pyridin-2-yl]-1,2,3,4-tétrahydro-quinoxaline dans 9 mL de dichlorométhane. On ajoute à 0°C, 1,1 ml de triéthylamine. On additionne ensuite 0,304 g de triphosgène. Le milieu réactionnel est agité 30 minutes à 0°C puis à température ambiante pendant 3h. 0,89 mL de triéthylamine sont de nouveau ajoutés et 0,65 g de l'amide de l'acide *trans*-4-amino-adamantane-1-carboxylique sont ensuite additionnés. Pour une meilleure solubilité, on ajoute 23 mL de diméthylformamide. La solution est agitée à température ambiante pendant 18h et est ensuite versée sur l'eau et extraite deux fois au dichlorométhane. Les phases organiques sont combinées et lavée deux fois à l'eau, séchée sur sulfate de magnésium, filtrée et évaporée à sec. Le brut obtenu est chromatographié sur gel de silice en éluant avec un gradient de méthanol dans le dichlorométhane variant de 1% à 10%. On obtient donc 0.428 g de *trans*-(5-carbamoyl-adamantan-2-yl)-amide de l'acide 4-[5-(1,1-difluoro-6-aza-spiro[2.5]oct-6-yl)-pyridin-2-yl]-3,4-dihydro-2H-quinoxaline-1-carboxylique que l'on dissous ensuite dans 4 mL de dichlorométhane, auquel sont ajoutés, sous agitation, 3,5 mL d'une solution 0.2N d'acide chlorhydrique dans l'éther éthylique. On évapore à sec et on reprend par de l'acétate d'éthyle. On obtient un précipité que l'on essore et sèche sous vide à 40°C. On obtient 0,37g de chlorhydrate du *trans*-(5-carbamoyl-adamantan-2-yl)-amide de l'acide 4-[5-(1,1-difluoro-6-aza-spiro[2.5]oct-6-yl)-pyridin-2-yl]-3,4-dihydro-2H-quinoxaline-1-carboxylique.
M+H⁺=577 ; PF=134-170°C ;
1 H NMR (400 MHz, DMSO-d6) δ(ppm) = 8,09 (singulet large, 1 H), 7,77 (m, 1 H), 7,53 (m, 1 H), 7,26 (m, 1H), 7,19 (m, 1H), 6,99 (m, 3H), 6,69 (singulet large, 1H), 6,16 (d, J = 5,8 Hz, 1 H), 3,86 (m, 4H), 3,74 (m, 1H), 3,30 (m, 4H), 2,00 (m, 3H), 1,94 to 1,64 (m, 12H), 1,45 (m, 2H), 1,38 (m, 2H)

### Exemple 5 Acide trans-6-{6-[4-(5-carbamoyl-adamantan-2-ylcarbamoyl)-3,4-dihydro-2H-quinoxalin-1-yl]-pyridin-3-yl}-6-aza-spiro[2.5]octane-1-carboxylique (composé n°12)

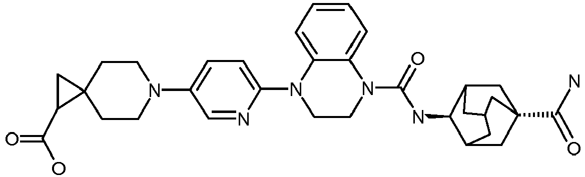

### 5.1 : 6'-(3,4-Dihydro-2H-quinoxalin-1-yl)-2,3,5,6-tétrahydro-[1,3']-bipyridinyl-4-one

Dans un ballon de 150 ml, on place 5,51 g de l'ester *tert*-butylique de l'acide 4-[5-(1,4-dioxa-8-aza-spiro[4.5]dec-8-yl)-pyridin-2-yl]-3,4-dihydro-2H-quinoxaline-1 -carboxylique (intermédiaire 7.1) dans 21 ml de tétrahydrofurane, 21 ml d'eau et 20 ml d'acétone. On refroidit au bain de glace et on ajoute doucement 8,24 ml d'acide sulfurique 95%. On laisse agiter à température ambiante pendant 18h. La solution est alors versée sur l'eau. On ajoute de l'hydroxyde de sodium 5N jusqu'à pH basique et on extrait deux fois à l'acétate d'éthyle. Les phases organiques réunies sont lavées à l'eau, séchées sur sulfate de sodium, filtrées et évaporées à sec. Le brut obtenu est chromatographié sur gel de silice en éluant avec un gradient de solvants ternaires, d'un côté l'heptane et de l'autre heptane/acétate d'éthyle/méthanol 4/5/1 variant de 10% à 100%. On obtient 2,39 g de 6'-(3,4-dihydro-2H-quinoxalin-1-yl)-2,3,5,6-tétrahydro-[1,3']bipyridinyl-4-one.
M+H⁺=309

### 5. 2 : Ester éthylique de l'acide [6'-(3,4-dihydro-2H-quinoxalin-1-yl)-2,3,5,6-tétrahydro-[1,3']bipyridinyl-4-ylidène]-acétique

Dans un ballon de 100 ml, on introduit 1,91 g de triéthylphosphonoacétate et 19 mL de tétrahydrofurane anhydre. On refroidit au bain de glace. A +5°C, on ajoute à la spatule petit à petit 0,205 g d'hydrure de sodium 95%. Une fois l'ajout terminé, on remonte à température ambiante et on agite le milieu réactionnel pendant 30 minutes. Puis on plonge le ballon dans un bain de glace et on ajoute à +5°C 0,205 g d'hydrure de sodium 95%. On agite pendant 30 minutes à température ambiante. Dans un autre ballon de 100 ml, on introduit 2,39 g de 6'-(3,4-dihydro-2H-quinoxalin-1-yl)-2,3,5,6-tétrahydro-[1,3']bipyridinyl-4-one et 19 mL de tétrahydrofurane anhydre. A +5°C de nouveau, on ajoute la solution d'ylure à la solution de 6'-(3,4-dihydro-2H-quinoxalin-1-yl)-2,3,5,6-tétrahydro-[1,3']bipyridinyl-4-one. On remonte à température ambiante et on agite pendant 18h. On verse le milieu réactionnel sur l'eau et on extrait trois fois à l'acétate d'éthyle. Les phases organiques sont lavées à l'eau, séchées sur sulfate de magnésium, filtrées et évaporées à sec. Le brut obtenu est d'abord chromatographié sur gel de silice en éluant avec un gradient de solvants ternaires, d'un côté l'heptane et de l'autre heptane/acétate d'éthyle/méthanol 4/5/1 variant de 10% à 100%. Le produit obtenu est de nouveau chromatographié sur gel de silice avec un gradient de méthanol dans le dichlorométhane variant de 1% à 10%. On obtient 0,468 g de l'ester éthylique de l'acide [6'-(3,4-dihydro-2H-quinoxalin-1-yl)-2,3,5,6-tétrahydro-[1,3']bipyridinyl-4-ylidène]-acétique. La phase aqueuse, quant à elle, contient l'acide [6'-(3,4-dihydro-2H-quinoxalin-1-yl)-2,3,5,6-tétrahydro-[1,3']bipyridinyl-4-ylidène]-acétique que l'on récupère en ajoutant une solution saturée d'acide sulfureux dans l'eau et en extrayant au dichlorométhane et acétate d'éthyle. La phase organique ainsi obtenue est séchée sur sulfate de sodium, filtrée et évaporée à sec. On obtient 1,08 g de l'acide [6'-(3,4-dihydro-2H-quinoxalin-1-yl)-2,3,5,6-tétrahydro-[1,3']bipyridinyl-4-ylidène]-acétique. Ces 1,08 g d'acide sont alors réestérifiés à l'aide de 0,90 ml d'acide sulfurique dans 15 ml d'éthanol à reflux pendant 3h. Le milieu réactionnel est alors versé sur de l'eau + glace. On ajoute de l'hydrogénocarbonate de sodium jusqu'à pH 8. On extrait deux fois à l'acétate d'éthyle, on lave à l'eau et à l'eau saturée en chlorure de sodium. On sèche sur sulfate de magnésium, on filtre et on évapore à sec. Le brut obtenu est chromatographié sur gel de silice en éluant avec un gradient de méthanol dans le dichlorométhane variant de 1% à 10%. On obtient 0,736 g de l'ester éthylique de l'acide [6'-(3,4-dihydro-2H-quinoxalin-1-yl)-2,3,5,6-tétrahydro-[1,3']bipyridinyl-4-ylidène]-acétique La quantité totale obtenue est donc 1,2 g de l'ester éthylique de l'acide [6'-(3,4-dihydro-2H-quinoxalin-1-yl)-2,3,5,6-tétrahydro-[1,3']bipyridinyl-4-ylidène]-acétique.
M+H⁺=379

### 5. 3 : Ester éthylique de l'acide 6-[6-(3,4-dihydro-2H-quinoxalin-1-yl)-pyridin-3-yl]-6-aza-spiro[2.5]octane-1-carboxylique

Dans un ballon de 150 ml, on on introduit 1,04 g d'iodure de triméthylsulfoxonium dans 15 mL de diméthylsulfoxide. On ajoute à température ambiante 0,533 g de tertbutylate de potassium. On laisse agiter à température ambiante pendant 3h. On ajoute une solution de 1,2 g de l'ester éthylique de l'acide [6'-(3,4-dihydro-2H-quinoxalin-1-yl)-2,3,5,6-tétrahydro-[1,3']bipyridinyl-4-ylidène]-acétique dissous dans 15 ml de diméthylsulfoxide. On agite à température ambiante pendant 3h et on laisse au repos pendant 2 jours. On prépare de nouveau de l'iodure de triméthylsulfoxonium (0,78 g) dans 8 mL de diméthylsulfoxyde, solution à laquelle on ajoute 0,4 g de tertbutylate de potassium. On laisse agiter 3h. Ensuite, on ajoute cette solution au milieu réactionnel et on agite à température ambiante pendant 18h. La solution est alors versée sur de l'eau et extraite trois à l'acétate d'éthyle. Les phases organiques réunies sont séchées sur sulfate de sodium, filtrées et évaporées à sec. Le brut obtenu est chromatographié sur gel de silice en éluant avec un gradient de méthanol dans le dichlorométhane variant de 0.5% à 5%. Le produit est de nouveau chromatographié sur gel de silice en éluant avec un gradient de solvants ternaires, d'un côté le dichlorométhane et de l'autre dichlorométhane/acétate d'éthyle/méthanol 70/25/5 variant de 10% à 100%. On obtient 0,338 g de l'ester éthylique de l'acide 6-[6-(3,4-dihydro-2H-quinoxalin-1-yl)-pyridin-3-yl]-6-aza-spiro[2.5]octane-1-carboxylique.
M+H⁺=393

### 5.4 : Ester éthylique de l'acide trans-6-{6-[4-(5-carbamoyl-adamantan-2-ylcarbamoyl)-3,4-dihydro-2H-quinoxalin-1-yl]-pyridin-3-yl}-6-aza-spiro[2.5]octane-1-carboxylique

Dans un tricol de 50 ml sous atmosphère inerte d'azote, on introduit 0,338 g d'ester éthylique de l'acide 6-[6-(3,4-dihydro-2H-quinoxalin-1-yl)-pyridin-3-yl]-6-aza-spiro[2.5]octane-1-carboxylique dans 8 mL de dichlorométhane. On ajoute à 0°C, 0,36 mL de triéthylamine. On additionne ensuite à 0°C, 0,102 g de triphosgène. Le milieu réactionnel est agité 30 minutes à 0°C puis à température ambiante pendant 3h. 0,30 mL de triéthylamine sont de nouveau ajoutés et 0,22 g de l'amide de l'acide *trans-4-*amino-adamantane-1-carboxylique sont ensuite additionnés. Pour une meilleure solubilité, on ajoute 8 mL de diméthylformamide. La solution est agitée à température ambiante pendant 18h. Elle est ensuite versée sur l'eau et extraite deux fois au dichlorométhane. Les phases organiques sont combinées et lavées trois fois à l'eau, séchées sur sulfate de magnésium, filtrées et évaporées à sec. Le brut obtenu est chromatographié sur gel de silice en éluant avec un gradient de solvants ternaires, d'un côté l'heptane et de l'autre heptane/acétate d'éthyle/méthanol 4/5/1 variant de 10% à 100%. On obtient 0,343 g de l'ester éthylique de l'acide *trans*-6-{6-[4-(5-carbamoyl-adamantan-2-ylcarbamoyl)-3,4-dihydro-2H-quinoxalin-1-yl]-pyridin-3-yl}-6-aza-spiro[2.5]octane-1-carboxylique.
M+H⁺=613

### 5.5: Acide trans-6-{6-[4-(5-carbamoyl-adamantan-2-ylcarbamoyl)-3,4-dihydro-2H-quinoxalin-1-yl]-pyridin-3-yl}-6-aza-spiro[2.5]octane-1-carboxylique

Dans un ballon de 50ml, on place 0,343 g de l'ester éthylique de l'acide *trans-6-*{6-[4-(5-carbamoyl-adamantan-2-ylcarbamoyl)-3,4-dihydro-2H-quinoxalin-1-yl]-pyridin-3-yl}-6-aza-spiro[2.5]octane-1-carboxylique dans 6 ml d'un mélange 1/1/1 de tétrahydrofurane/ méthanol/ eau. On ajoute à température ambiante 0,090 g d'hydroxyde de lithium monohydrate. On agite à température ambiante pendant 18h. On évapore à sec. On reprend par de l'eau et on ajoute une solution saturée d'acide sulfureux dans l'eau jusqu'à pH acide. Un précipité se forme. Il est essoré et séché sous vide à 40°C. On obtient 0,214 g de l'acide *trans*-6-{6-[4-(5-carbamoyl-adamantan-2-ylcarbamoyl)-3,4-dihydro-2H-quinoxalin-1-yl]-pyridin-3-yl}-6-aza-spiro[2.5]octane-1-carboxylique.
M+H⁺=585 ; PF= 145-160°C ;
1H NMR (400 MHz, DMSO-d6) δ(ppm) = 12,09 (m, 1H), 8,04 (d, J = 3 Hz, 1H), 7,41 (m, 2H), 7,09 (m, 2H), 7,00 à 6,80 (m, 3H), 6,68 (m, 1 H), 6,05 (d, J = 6 Hz, 1 H), 3,94 à 3,68 (m, 5H), 3,42 à 2,92 (m, 4H), 2,05 à 1,37 (m, 18H), 0,96 (m, 2H)

### Exemple 6 : trans-(5-carbamoyl-adamantan-2-yl)-amide de l'acide 4-[5-(4-méthyl-4-phényl-[1,4]azasilinan-1-yl)-pyridin-2-yl]-3,4-dihydro-2H-quinoxaline-1-carboxylique (composé n°13)

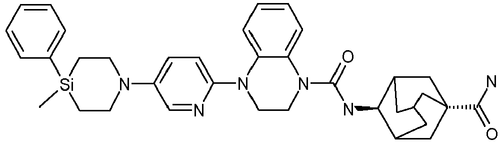

### 6.1 : Méthyl-phényl-divinyl-silane

158 ml d'une solution de chlorure de vinylmagnésium dans du THF est additionnée goutte à goutte à l'aide d'une ampoule d'addition sur 1h à température ambiante sous azote à 13,1 ml d'une solution de diméthoxyméthylphényldivinylsilane dans 36 ml de THF anhydre. La température est contrôlée par un bain d'eau. Après l'addition, le mélange est agité à température ambiante pendant 16h puis porté à reflux pendant 2h. 40 ml d'H₂O sont ensuite ajoutés et le milieu est agité 30 min. Le précipité blanc ainsi formé est ensuite filtré puis rincé avec de l'acétate d'éthyle. Le filtrat est extrait à l'acétate d'éthyle deux fois. Les phases organiques sont rassemblées, lavées avec une solution aqueuse saturée de chlorure de sodium, séchées sur sulfate de magnésium et concentrées sous vide. On obtient 12,56 g de méthyl-phényl-divinyl-silane sous forme d'une huile jaune claire qui est utilisé ensuite sans autre purification.
RMN ¹H (CDCl₃, 200MHz), δ(ppm) = 0,45 (s, 3H) ; 5,80 (dd, J= 19,6 et 4,5 Hz, 2H) ; 6,14 (dd, J= 14,6 et 4,5 Hz, 2H) ; 6,34 (dd, J= 19,6 et 14,6 Hz, 2H) ; 7,40-7,27 (m, 3H) ; 7,58-7,53 (m, 2H).

### 6.2 : Alcool 2-[(2-hydroxy-éthyl)-méthyl-phényl-silanyl]-éthylique

On place 16,1 g de méthyl-phényl-divinyl-silane dans 65 ml de THF anhydre sous azote à température ambiante. On ajoute 24,6 g du dimère 9-BBN et le mélange est porté à reflux pendant 4h. Une fois revenu à température ambiante, on ajoute 40 ml d'H₂O suivi de 90 ml d'une solution de soude à 3N. Le mélange est refroidi à 0°C et 90 ml d'une solution d'eau oxygénée à 30% sont précautionneusement ajoutés. Le mélange est ensuite porté à reflux pendant 2h. Le mélange biphasique est extrait à l'acétate d'éthyle trois fois. Les phases organiques sont rassemblées, lavées avec une solution aqueuse saturée de chlorure de sodium, séchées sur sulfate de magnésium et concentrées sous vide. Le brut obtenu est chromatographié sur gel de silice en éluant avec un gradient d'éthanol et de dichlorométhane dans l'heptane variant de 0,4/3,6/6 à 0,4/4,4/5. On obtient 15,1 g de l'alcool 2-[(2-hydroxy-éthyl)-méthyl-phényl-silanyl]-éthylique sous forme d'un solide blanc.
RMN ¹H (CDCl₃, 200MHz), δ(ppm) = 0,36 (s, 3H) ; 1,27 (t, J= 7,5 Hz, 4H) ; 2,11 (s large, 2H) ; 3,80 (t, J= 7,5 Hz, 4H) ; 7,40-7,36 (m, 3H) ; 7,55-7,50 (m, 2H).

### 6.3 : 1-Benzyl-4-méthyl-4-phényl-[1,4]azasilinane

On place 8,5 g de l'alcool 2-[(2-hydroxy-éthyl)-méthyl-phényl-silanyl]-éthylique dans 80 ml de dichlorométhane anhydre sous azote. On ajoute 14,2 ml de triéthylamine, puis à 0°C, 6,9 ml de chlorure de mésyle goutte à goutte à l'aide d'une ampoule d'addition. Le mélange est agité 2h à 0°C et devient progressivement orange avec apparition d'un précipité. Une fois la conversion totale, on rajoute 14,2 ml de triéthylamine puis 4,7 ml de benzylamine à 0°C. Le milieu réactionnel est ramené à température ambiante puis porté à reflux pendant 6h. Après hydrolyse avec H₂O à température ambiante, le milieu réactionnel est extrait à l'acétate d'éthyle trois fois. Les phases organiques sont rassemblées, lavées avec une solution aqueuse saturée de chlorure de sodium, séchées sur sulfate de magnésium et concentrées sous vide. Le brut obtenu est chromatographié sur gel de silice en éluant avec un gradient d'acétate d'éthyle et d'heptane contenant 3% de triéthylamine variant de 0/10 à 3/7. On obtient 3,6 g de 1-benzyl-4-méthyl-4-phényl-[1,4]azasilinane sous forme d'une huile jaune claire.
RMN ¹H (CDCl₃, 200MHz), δ(ppm) : 0,32 (s, 3H) ; 1,00-0,86 (m, 2H) ; 1,35-1,15 (m, 2H) ; 2,82 (s large, 4H) ; 3,62 (s large, 2H) ; 7,40-7,27 (m, 8H) ; 7,59-7,54 (m, 2H).

### 6.4 : Chlorhydrate de 4-méthyl-4-phényl-[1,4]azasilinane

On place 3,6g de 1-Benzyl-4-méthyl-4-phényl-[1,4]azasilinane dans 32 ml de dichlorométhane anhydre sous azote. A 0°C, 2,5 ml de chloroformiate de 1-chloroéthyle sont ajoutés goute à goutte au milieu réactionnel. Le mélange réactionnel est progressivement ramené à température ambiante puis agité 2h à reflux. Après conversion totale, le mélange réactionnel est concentré sous vide et séché puis le résidu est dissous dans 50 ml de méthanol à température ambiante. Le mélange est agité à reflux pendant 2h. Après évaporation du solvant sous vide, le solide jaune obtenu est ensuite suspendu dans l'acétate d'éthyle. La suspension est portée à reflux quelques minutes puis refroidie progressivement jusqu'à température ambiante. Les cristaux ainsi formés sont filtrés, lavés avec de l'acétate d'éthyle et séchés sous vide. On obtient 2,13g de chlorhydrate de 4-méthyl-4-phényl-[1,4]azasilinane sous forme de cristaux blancs.
PF= 245°C ; M+H⁺= 191 ;
RMN ¹H (DMSO, 200MHz), δ(ppm) = 0,40 (s, 3H) ; 1,11 (dt, J= 15,1 et 5,4 Hz, 2H) ; 1,33 (dd, J = 15,1 et 6,7 Hz, 2H) ; 3,31-3,23 (m, 4H) ; 7,46-7,38 (m, 3H) ; 7,62-7,57 (m, 2H) ; 8,79 (s large, 2H).

### 6.5 : Ester tert-butylique de l'acide 4-[5-(4-méthyl-4-phényl-[1,4]azasilinan-1-yl)-pyridin-2-yl]-3,4-dihydro-2H-quinoxaline-1-carboxylique

On place 0,5 g de l'ester *tert*-butylique de l'acide 4-(5-Bromo-pyridin-2-yl)-3,4-dihydro-2H-quinoxaline-1-carboxylique (intermédiaire 1.1) dans 6,5 ml de toluène puis on ajoute 0,21 g de 2-dicyclohexylphosphino-2',6'-diméthoxybiphényl, 0,117 g de tris(dibenzylidéneacétone)dipalladium (0), 0,307 g de *tert*-butylate de sodium et 0,321 g de chlorhydrate de 4-méthyl-4-phényl-[1,4]azasilinane. Le milieu réactionnel est chauffé à 105-110 °C pendant 19 h. Après concentration, on ajoute de l'acétate d'éthyle et le mélange est lavé avec une solution aqueuse de chlorure de sodium puis par de l'eau. La phase organique est séchée sur du sulfate de sodium et concentrée sous pression réduite. Le brut obtenu est chromatographié sur gel de silice en éluant avec gradient de solvants heptane / acétate d'éthyle (100/0 à 70/30). On obtient 0,43g de l'ester *tert*-butylique de l'acide 4-[5-(4-méthyl-4-phényl-[1,4]azasilinan-1-yl)-pyridin-2-yl]-3,4-dihydro-2H-quinoxaline-1-carboxylique.
M+H⁺ = 501,1

### 6.6 : 1-[5-(4-Méthyl-4-phényl-[1,4]azasilinan-1-yl)-pyridin-2-yl]-1,2,3,4-tétrahydroquinoxaline :

On place 0.43g de l'ester *tert*-butylique de l'acide 4-[5-(4-méthyl-4-phényl-[1,4]azasilinan-1-yl)-pyridin-2-yl]-3,4-dihydro-2H-quinoxaline-1-carboxylique dans 50 mL de dichlorométhane et après refroidissement au bain de glace on ajoute 3,5 mL d'une solution 4 N d'acide chlorhydrique dans le dioxanne. Le milieu réactionnel est agité pendant 16h. Après concentration le milieu est repris dans du dichlorométhane, neutralisé par ajout d'une solution saturée de bicarbonate de sodium. Après décantation la phase aqueuse est extraite par du dichlorométhane. Les phases dichlorométhane sont regroupées et séchées sur du sulfate de sodium. Après concentration sous vide on obtient 0,338 g de 1-[5-(4-méthyl-4-phényl-[1,4]azasilinan-1-yl)-pyridin-2-yl]-1,2,3,4-tétrahydro-quinoxaline.
M+H⁺ = 401,1

### 6.7 : trans-(5-Carbamoyl-adamantan-2-yl)-amide de l'acide 4-[5-(4-méthyl-4-phényl-[1,4]azasilinan-1-yl)-pyridin-2-yl]-3,4-dihydro-2H-quinoxaline-1-carboxylique

Dans un tricol sous atmosphère d'azote on place 0.338g de 1-[5-(4-méthyl-4-phényl-[1,4]azasilinan-1-yl)-pyridin-2-yl]-1,2,3,4-tétrahydro-quinoxaline et 15 ml de dichlorométhane. On ajoute 0,35 mL de triéthylamine. Le milieu est refroidi par un mélange glace/acétone à - 8 °C puis on ajoute 0,133 g de triphosgène. Le milieu réactionnel est ensuite agité à température ambiante pendant 3 h. Dans un ballon on place 10 ml de diméthylformamide, 0,193 g de chlorhydrate de l'amide de l'acide trans-4-amino-adamantane-1-carboxylique et 0,35 ml de triéthylamine. Le mélange est placé dans une cuve à ultra-sons puis chauffé pour obtenir un mélange quasi limpide. Ce mélange est additionné par portions au chlorure de carbamoyle précédemment préparé qui est refroidi par un bain d'eau. Le mélange réactionnel est agité à température ambiante pendant 16 h. Après évaporation du dichlorométhane le milieu réactionnel est repris dans 300 ml d'acétate d'éthyle et lavé par une solution saturée de chlorure de sodium. La phase organique est séchée sur du sulfate de sodium puis concentrée sous vide. Le brut obtenu est chromatographié sur gel de silice en éluant avec un gradient de solvants dichlorométhane / méthanol (100/0 à 90/10). On obtient 0,28 g de *trans*-(5-carbamoyl-adamantan-2-yl)-amide de l'acide 4-[5-(4-méthyl-4-phényl-[1,4]azasilinan-1-yl)-pyridin-2-yl]-3,4-dihydro-2H-quinoxaline-1-carboxylique.
M+H+= 621 ; PF=106-108°C ;
1 H NMR (400 MHz, DMSO-d6) δ(ppm) = 8,05 (d, J = 3.1 Hz, 1 H), 7,52 (m, 2H), 7,43 à 7,31 (m, 5H), 7,11 (d, J = 9Hz, 1 H), 7,03 (dd, J = 8.1 Hz et 1,5 Hz, 1 H), 6,98 (m, 1 H), 6,92 (m, 1 H), 6,81 (m, 1 H), 6,69 (m, 1 H), 6,03 (d, J = 6,2 Hz, 1 H), 3,83 à 3,61 (m, 9H), 2,02 à 1,69 (m, 11H), 1,46 (m, 2H), 1,12 (m, 2H), 0,94 (m, 2H), 0,35 (s, 3H)

### Exemple 7 : Chlorhydrate du trans-(5-carbamoyl-adamantan-2-yl)-amide de l'acide 4-[4-((R)-3-hydroxy-pyrrolidin-1-yl)-3,4,5,6-tétrahydro-2H-[1,3']bipyridinyl-6'-yl]-3,4-dihydro-2H-quinoxaline-1-carboxylique (composé n° 9)

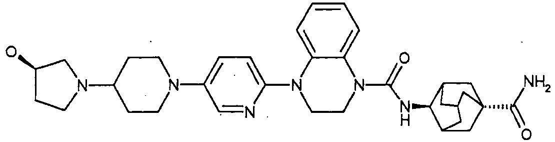

### 7.1 : Ester tert-butylique de l'acide 4-[5-(1,4-dioxa-8-aza-spiro[4.5]dec-8-yl)-pyridin-2-yl]-3,4-dihydro-2H-quinoxaline-1-carboxylique

On place 2 g de l'ester *tert*-butylique de l'acide 4-(5-bromo-pyridin-2-yl)-3,4-dihydro-2H-quinoxaline-1-carboxylique (intermédiaire 1.1) dans 25 ml de toluène. On ajoute 0,81 g de 1,4-dioxa-8-aza-spiro[4.5]décane, 0,69 g de *tert*-butoxyde de sodium, 0,34 g de 2-dicyclohexylphosphino-2',6'-diméthoxybiphényl et 0,19 g de tris(dibenzylidèneacétone)dipalladium (0). Le milieu réactionnel est agité 2h sous azote à 110°C. Le milieu réactionnel est versé dans 200 ml d'H₂O, extrait 2 fois par 150 ml d'acétate d'éthyle. Les phases organiques sont combinées, lavées 2 fois par 100 ml d'H₂O, puis par 100 ml d'une solution aqueuse saturée en chlorure de sodium, séchées sur MgSO₄ et concentrées à sec. Le brut obtenu est chromatographié sur gel de silice en éluant avec un gradient d'acétate d'éthyle dans de l'heptane, variant de 5% à 50%. On obtient 2,4 g de l'ester *tert*-butylique de l'acide 4-[5-(1,4-dioxa-8-aza-spiro[4.5]dec-8-yl)-pyridin-2-yl]-3,4-dihydro-2H-quinoxaline-1-carboxylique.
[M+H⁺] = 453

### 7.2 : 1-[5-(1,4-Dioxa-8-aza-spiro[4.5]déc-8-yl)-pyridin-2-yl]-1,2,3,4-tétrahydro-quinoxaline

On place 2,7 g de l'ester *tert*-butylique de l'acide 4-[5-(1,4-dioxa-8-aza-spiro[4.5]dec-8-yl)-pyridin-2-yl]-3,4-dihydro-2H-quinoxaline-1-carboxylique dans 30 ml de dioxane, puis on ajoute 22,37 ml d'HCl 4N dans le dioxane. Le milieu réactionnel est agité 48h en milieu clos à température ambiante. Après concentration à sec, le milieu réactionnel est dilué par 200 ml d'une solution aqueuse saturée d'hydrogénocarbonate de sodium et extrait 2 fois par 200 ml de dichlorométhane. Les phases organiques résultantes sont combinées, lavées par 100 ml d'une solution aqueuse saturée d'hydrogénocarbonate de sodium, 100 ml d'une solution aqueuse saturée en chlorure de sodium, séchées sur MgSO₄ et concentrées à sec. On obtient 2,1 g de 1-[5-(1,4-dioxa-8-aza-spiro[4.5]dec-8-yl)-pyridin-2-yl]-1,2,3,4-tétrahydro-quinoxaline.
[M+H⁺] = 353

### 7.3 : trans-(5-Carbamoyl-adamantan-2-yl)-amide de l'acide 4-[5-(1,4-dioxa-8-azaspiro[4.5]dec-8-yl)-pyridin-2-yl]-3,4-dihydro-2H-quinoxaline-1-carboxylique

On place 2,25 g de 1-[5-(1,4-dioxa-8-aza-spiro[4.5]dec-8-yl)-pyridin-2-yl]-1,2,3,4-tétrahydro-quinoxaline dans 30 mL de dichlorométhane à 0°C. On ajoute 1,78 mL de triéthylamine puis 0,76 g de triphosgène. Le milieu réactionnel est agité 30 min sous azote à 0°C, puis 3 heures à température ambiante. On ajoute alors 1,47 g du chlorhydrate de l'amide de l'acide *trans*-4-amino-adamantane-1-carboxylique, 2,22 ml de triéthylamine et 30 ml de DMF. Le milieu réactionnel est agité 18h à température ambiante sous azote. Après hydrolyse avec 250 ml d'H₂O, le milieu est extrait 2 fois par 350 mL de dichlorométhane. Les phases organiques résultantes sont combinées, lavées 2 fois par 200 ml d'H₂O, puis par 200ml d'une solution aqueuse saturée en chlorure de sodium, séchées sur MgSO₄ et concentrées à sec. Le brut obtenu est chromatographié 2 fois sur gel de silice en éluant avec un gradient de méthanol dans du dichlorométhane variant de 1% à 10%. On obtient 2,3 g de *trans*-(5-carbamoyl-adamantan-2-yl)-amide de l'acide 4-[5-(1,4-dioxa-8-aza-spiro[4.5]dec-8-yl)-pyridin-2-yl]-3,4-dihydro-2H-quinoxaline-1-carboxylique.
[M+H⁺] = 573 ; Pf = 177°C

### 7.4 : trans-(5-Carbamoyl-adamantan-2-yl)-amide de l'acide 4-(4-oxo-3,4,5,6-tétrahydro-2H-[1,3']bipyridinyl-6'-yl)-3,4-dihydro-2H-quinoxaline-1-carboxylique

On place 2,1 g de *trans*-(5-carbamoyl-adamantan-2-yl)-amide de l'acide 4-[5-(1,4-dioxa-8-aza-spiro[4.5]dec-8-yl)-pyridin-2-yl]-3,4-dihydro-2H-quinoxaline-1-carboxylique dans 40 ml d'un mélange 1/1 acétone/eau. On ajoute lentement 1,17 ml d'acide sulfurique concentré. Le milieu réactionnel est agité 48h sous azote à température ambiante. Le milieu réactionnel est concentré (évaporation de l'acétone), basifié à 0°C par une solution aqueuse d'hydroxyde de sodium 1 M jusqu'à pH 10 puis extrait 3 fois par 200 ml de dichlorométhane. Les phases organiques résultantes sont combinées, lavées par 200 mL d'H₂O, puis par 200mL d'une solution aqueuse saturée en chlorure de sodium, séchées sur MgSO₄ et concentrées à sec. On obtient 1,6 g *trans*-(5-carbamoyl-adamantan-2-yl)-amide de l'acide 4-(4-oxo-3,4,5,6-tétrahydro-2H-[1,3']bipyridinyl-6'-yl)-3,4-dihydro-2H-quinoxaline-1-carboxylique.
[M+H⁺] = 529

### 7.5 : trans-(5-Carbamoyl-adamantan-2-yl)-amide de l'acide 4-[4-((R)-3-hydroxy-pyrrolidin-1-yl)-3,4,5,6-tétrahydro-2H-[1,3']bipyridinyl-6'-yl]-3,4-dihydro-2H-quinoxaline-1-carboxylique

On place 0,2 g de *trans*-(5-carbamoyl-adamantan-2-yl)-amide de l'acide 4-(4-oxo-3,4,5,6-tétrahydro-2H-[1,3']bipyridinyl-6'-yl)-3,4-dihydro-2H-quinoxaline-1-carboxylique dans 2 ml de dichlorométhane. On ajoute 0,034 g de l'alcool (*R*)-pyrrolidin-3-ol puis 0,096 g de triacétoxyborohydrure de sodium. Le milieu réactionnel est agité 18h sous azote à température ambiante. Le milieu réactionnel est dilué par 75 ml de dichlorométhane, lavé par 75 ml d'une solution aqueuse saturée d'hydrogénocarbonate de sodium. La phase aqueuse est extraite par 75 ml de dichlorométhane et les phases organiques résultantes sont combinées, lavées par 50 mL d'une solution aqueuse saturée d'hydrogénocarbonate de sodium, 50 ml d'une solution aqueuse saturée en chlorure de sodium, séchées sur MgSO₄ et concentrées à sec. Le brut obtenu est chromatographié sur gel de silice en éluant avec un mélange de 10% de méthanol dans le dichlorométhane puis avec un mélange de 2% d'ammoniaque et de 20% de méthanol dans le dichlorométhane. On obtient 0,19 g de *trans*-(5-carbamoyl-adamantan-2-yl)-amide de l'acide 4-[4-((R)-3-hydroxy-pyrrolidin-1-yl)-3,4,5,6-tétrahydro-2H-[1,3']bipyridinyl-6'-yl]-3,4-dihydro-2H-quinoxaline-1-carboxylique.
[M+H⁺] = 600

### 7.6 : Chlorhydrate du trans-(5-carbamoyl-adamantan-2-yl)-amide de l'acide 4-[4-((R)-3-hydroxy-pyrrolidin-1-yl)-3,4,5,6-tétrahydro-2H-[1,3']bipyridinyl-6'-yl]-3,4-dihydro-2H-quinoxaline-1-carboxylique

On place le *trans*-(5-carbamoyl-adamantan-2-yl)-amide de l'acide 4-[4-((R)-3-hydroxypyrrolidin-1-yl)-3,4,5,6-tétrahydro-2H-[1,3']bipyridinyl-6'-yl]-3,4-dihydro-2H-quinoxaline-1-carboxylique (0,19 g, 0,32 mmol) dans 5 mL de dichlorométhane. On ajoute 1,6 mL d'une solution d'acide chlorhydrique 0.2 M dans l'éther diéthylique. Le milieu réactionnel est concentré à sec, repris par 5 mL d'acétate d'éthyle, trituré puis filtré et séché sous vide à 40°C pendant 18h. On obtient 0,18g de chlorhydrate du *trans*-(5-carbamoyl-adamantan-2-yl)-amide de l'acide 4-[4-((*R*)-3-hydroxy-pyrrolidin-1-yl)-3,4,5,6-tétrahydro-2H-[1,3']bipyridinyl-6'-yl]-3,4-dihydro-2H-quinoxaline-1-carboxylique.
[M+H⁺] = 600 ; Pf = 191 °C ;
1H NMR (400 MHz, DMSO-d6) δ(ppm) = 11,12 (m, 0,5H), 10,38 (m, 0,5H), 8,05 (m, 1 H), 7,54 (m, 1 H), 7,48 (m, 1H), 7,18 (m, 2H), 6,96 (m, 3H), 6,69 (s;large, 1H), 6,11 (m, 1H), 4,44 (m, 1H), 3,91 à 3,00 (m, 13H), 2,74 (m, 2H), 2,32 à 1,68 (m, 17H), 1,45 (m, 2H)

Le tableau qui suit illustre les structures chimiques et les propriétés physiques de quelques composés selon l'invention, répondant à la formule (I), et se présentant sous forme de bases libres ou de composés salifiés :
- dans la colonne « A », « - » représente une liaison simple ;
- base correspond à la molécule non salifiée
- dec. correspond à une température de décomposition ;
- HCl représente un chlorhydrate ;
- Me représente un groupe méthyle ;
- PF représente le point de fusion du composé, exprimé en degrés Celsius ;
- Sel correspond à la forme du composé qui peut être sous forme de la base ou sous forme salifié, par exemple un chlorhydrate ;
- M+H⁺ représente la masse du composé, obtenue par LC-MS (Liquid Chromatography Mass Spectroscopy).

**Tableau**

| | | | | | | | | | | | | | | | | |
|---|---|---|---|---|---|---|---|---|---|---|---|---|---|---|---|---|
| 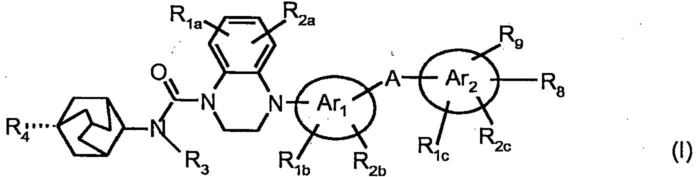 | | | | | | | | | | | | | | | | |

| **N°** | **A** | **R₁ₐ** | **R₂ₐ** | **R_{1b}** | **R_{1c}** | **R_{2c}** | **R₃** | **R₄** | **R₈** | **R₉** | **Ar₁** | **Ar₂** | **Sel** | **PF (°C)** | **M+H⁺** | **Synthèse** |
|---|---|---|---|---|---|---|---|---|---|---|---|---|---|---|---|---|
| **1** | - | H | H | H | H | H | H | OH *(trans)* | -CH₂Si(Me)₃ | H | 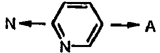 | 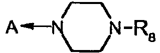 | | | | |
| **2** | - | H | H | H | H | H | H | OH *(trans)* | =N-O-C(Me)₃ | H | 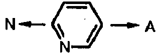 | 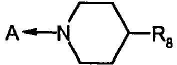 | Base | 110-113°C | 573 | Méthode 1 |
| **3** | - | H | H | H | H | H | H | OH *(trans)* | -SO₂(CH₂)₂Si(Me)₃ | H | 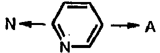 | 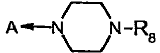 | | | | |
| **4** | - | H | H | H | H | H | H | -CONH₂ *(trans)* | 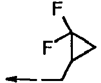 | H | 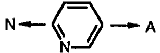 | 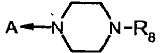 | Base | 206 | 606 | Méthode 1 |
| **5** | - | H | H | H | H | H | H | -CONH₂ *(trans)* | 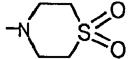 | H | 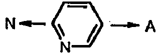 | 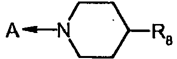 | Base | 160-200 | 648 | Méthode 1 |
| **6** | - | H | H | H | H | H | H | -CONH₂ (*trans)* | 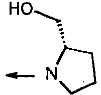 | H | 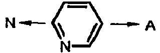 | 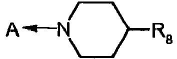 | | | | |
| **7** | - | H | H | H | H | H | H | -CONH₂ *(trans)* | 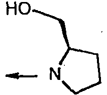 | H | 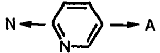 | 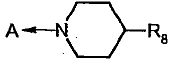 | | | | |
| **8** | - | H | H | H | H | H | H | -CONH₂ *(trans)* | 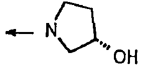 | H | 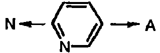 | 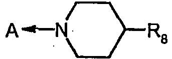 | | | | |
| **9** | - | H | H | H | H | H | H | -CONH₂ *(trans)* | 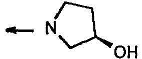 | H | 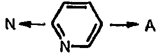 | 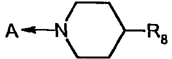 | HCl | 191 | 600 | Méthode 2 |
| **10** | - | H | H | H | H | H | H | -CONH₂ *(trans)* | 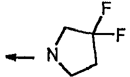 | H | 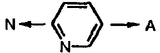 | 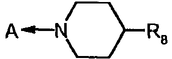 | | | | |
| **11** | - | H | H | H | H | H | H | -CONH₂ *(trans)* | 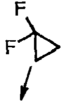 | | 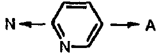 | 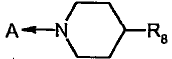 | HCl | 134-170 | 577 | Méthode 1 |
| **12** | - | H | H | H | H | H | H | -CONH₂ *(trans)* | 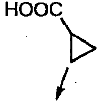 | | 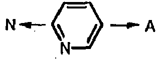 | 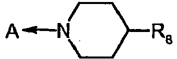 | Base | 145-160 | 585 | Méthode 1 |
| **13** | - | H | H | H | H | H | H | -CONH₂ *(trans)* | 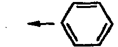 | Me | 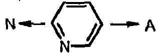 | 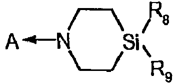 | Base | 106-108 | 621 | Méthode 1 |
| **14** | - | H | H | H | H | H | H | -CONH₂ *(trans)* | Me | Me | 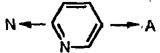 | 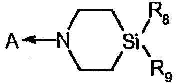 | | | | |

Les composés selon l'invention ont fait l'objet d'essais pharmacologiques permettant de déterminer leur effet inhibiteur de l'enzyme 11βHSD1, qui intervient dans le métabolisme des lipides et le métabolisme du glucose.

Ces essais ont consisté à mesurer l'activité inhibitrice *in vitro* des composés de l'invention sur l'enzyme 11βHSD1 grâce à un test SPA (Scintillation Proximity Assay) en format 384 puits, La protéine 11βHSD1 recombinante a été produite en levure *S.cerevisiae.* La réaction a été réalisée en incubant l'enzyme en présence de ³H-cortisone et de NADPH, en absence ou en présence de concentration croissante d'inhibiteur. Des billes SPA couplées à un anticorps anti-souris, préincubées avec un anticorps anti-cortisol, ont permis de mesurer la quantité de cortisol formé au cours de la réaction.

L'activité inhibitrice vis-à-vis de l'enzyme 11βHSD1 est donnée par la concentration qui inhibe 50% de l'activité de 11βHSD1 (Cl₅₀).

Les Cl₅₀ des composés de l'invention sont reportés dans le tableau ci-après :

| **Composé n°** | **11b/HSD1-HR Cl₅₀ nM** |
|---|---|
| **1** | **7** |
| **2** | **23** |
| **3** | **10** |
| **4** | **3** |
| **5** | **4** |
| **6** | **4** |
| **7** | **3** |
| **8** | **6** |
| **9** | **2** |
| **10** | **2** |
| **11** | **3** |
| **12** | **6** |
| **13** | **25** |
| **14** | **12** |

Il apparaît donc que les composés selon l'invention ont une activité inhibitrice de l'enzyme 11βHSD1. Les composés selon l'invention peuvent donc être utilisés pour la préparation de médicaments, en particulier de médicaments inhibiteurs de l'enzyme 11βHSD1.

Ainsi, selon un autre de ses aspects, l'invention a pour objet des médicaments qui comprennent un composé de formule (I), ou un sel d'addition de ce dernier à un acide ou à une base pharmaceutiquement acceptable, ou encore un hydrate ou un solvat du composé de formule (I).

Ces médicaments trouvent leur emploi en thérapeutique, notamment dans le traitement et la prévention de l'obésité, des diabètes, des troubles de la microcirculation, de la résistance à l'insuline, du syndrome métabolique, du syndrome de Cushing, de l'hypertension, de l'athérosclérose, de la cognition et de la démence, des glaucomes, de l'ostéoporose, de la lipodystrophie, de l'hypertrophie cardiaque, de l'insuffisance cardiaque, de maladies hépatiques, de certaines maladies infectieuses en augmentant l'efficacité du système immunitaire ou encore pour favoriser la cicatrisation de plaies.

Selon un autre de ses aspects, la présente invention concerne des compositions pharmaceutiques comprenant, en tant que principe actif, un composé selon l'invention, Ces compositions pharmaceutiques contiennent une dose efficace d'au moins un composé selon l'invention ou un sel pharmaceutiquement acceptable, un hydrate ou solvat dudit composé, ainsi qu'au moins un excipient pharmaceutiquement acceptable, Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale, telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants, Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) : 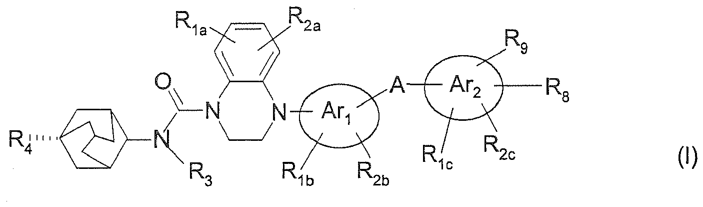 dans laquelle :
- A représente une liaison, un atome d'oxygène ou un groupe -O-CH₂-,
- Ar₁ représente un groupe phényle ou hétéroaryle,
- Ar₂ représente un groupe phényle, un groupe hétéroaryle ou un groupe hétérocycloalkyle,
- R_{1a,b,c} et R_{2a,b,c}, identiques ou différents, représentent chacun un atome d'hydrogène ou d'halogène ou un groupe alkyle ; cycloalkyle éventuellement substitué par un groupe alkyle, halogénoalkyle, alcoxy-alkyle, alcoxy-halogénoalkyle ou-COOR_{5;};-alkyl-cycloalkyle éventuellement substitué par un ou plusieurs atomes d'halogène ; -OR₅ (hydroxy ou alcoxy) ; hydroxy-alkyle ; alcoxy-alkyle ; alcoxy-alcoxy ; halogénoalkyle ; -O-halogénoalkyle ; oxo ; -CO-alkyle ; -CO-alkyl-NR₆R₇ ; -CO-halogénoalkyle ; -COOR₅ ; alkyl-COOR₅; -O-alkyl-COOR₅ ; -SO₂-alkyle ; -SO₂-cycloalkyle ; -SO₂-alkyl-cycloalkyle ; -SO₂-alkyl-OR₅ ; -SO₂-alkyl-COOR₅ ; -SO₂-alkyl-NR₆R₇ ; -SO₂-halogénoalkyle ; alkyl-SO₂-alkyle ; -SO₂-NR₆R₇ ; -SO₂-alkyl-alcoxy-alcoxy ; -CONR₆R₇; -alkyl-CONR₆R₇ ou -O-alkyl-NR₆R₇, ou encore R₁ₐ, R_{1b}, R_{1c} sont liés respectivement à R_{2a,} R_{2b}, R_{2c} et à l'atome de carbone qui les porte et représentent -O-alkyl-O- ;
- R₃ représente un atome d'hydrogène ou un groupe alkyle,
- R₄ représente un atome d'hydrogène ou d'halogène ou un groupe cyano, -OR₅, hydroxy-alkyle, -COOR₅, -NR₆R₇, -CONR₆R₇, -SO₂-alkyle ou -SO₂-NR₆R₇, -NR₆-COOR₅, -NR₆-COR₅, -CO-NR₆-alkyl-OR₅;
- R₅. R₆ et R₇, identiques ou différents, représentent chacun un atome d'hydrogène, un groupe alkyle ou un groupe -alkyl-phényle, et
- R₈ représente un groupe alkyle, alkyl-Si(alkyle)₃; -SO₂-alkyl-Si(alkyle)₃ ; phényle ; alcoxy-imino ; alkyle-cycloalkyle éventuellement substitué par un ou plusieurs atomes d'halogène; hétérocycloalkyle substitué par un ou plusieurs atomes d'halogène, un ou plusieurs groupes hydroxy ou hydroxy-alkyle ; ou bien R₈ et R₉ ensemble avec l'atome de carbone auquels ils sont liés forment un groupe cycloalkyle éventuellement substitué par un ou plusieurs atomes d'halogène ou un ou plusieurs groupes carboxy ;
- R₉ représente un atome d'hydrogène ou un groupe alkyle ;
à la condition que lorque R₈ est un groupe alkyle, il est fixé sur l'atome de silicium de Ar₂.
à l'état de base ou d'acide ou de sel d'addition à un acide ou à une base.

2. Composé de formule (I) selon la revendication 1, **caractérisé en ce que** A représente une liaison.

3. Composé de formule (I) selon l'une des revendications 1 ou 2, **caractérisé en ce que** Ar₁ représente un groupe hétéroaryle.

4. Composé de formule (I) selon la revendication 3, **caractérisé en ce que** Ar₁ représente le groupe pyridinyle.

5. Composé de formule (I) selon l'une quelconque des revendications 1 à 4, **caractérisé en ce que** Ar₂ représente un groupe hétérocycloalkyle.

6. Composé de formule (I) selon la revendication 5, **caractérisé en ce que** Ar₂ représente le groupe pipéridinyle, pipérazinyle ou azasilinanyle.

7. Composé de formule (I) selon l'une quelconque des revendications 1 à 6, **caractérisé en ce que** R₁ₐ, R₂ₐ, R_{1b} et R_{2b}, représentent chacun un atome d'hydrogène.

8. Composé de formule (I) selon l'une quelconque des revendications 1 à 7, **caractérisé en ce que** R₃ représente un atome d'hydrogène.

9. Composé de formule (I) selon l'une quelconque des revendications 1 à 8, **caractérisé en ce que** R₄ représente un groupe hydroxy ou -CONH₂.

10. Composé de formule (I) selon l'une quelconque des revendications 9, **caractérisé en ce que** R₈ représente un groupe alkyle, alkyl-Si(alkyle)₃; -SO₂-alkyl-Si(alkyle)₃ ; phényle ; alcoxy-imino ; hétérocycloalkyle substitué par un ou plusieurs atomes d'halogène, un ou plusieurs groupes hydroxy ou hydroxy-alkyle ; ou bien R₈ et R₉ ensemble avec l'atome de carbone auquels ils sont liés forment un groupe cycloalkyle éventuellement substitué par un ou plusieurs atomes d'halogène ou un ou plusieurs groupes carboxy ;
- R₉ représente un atome d'hydrogène ou un groupe alkyle ;
à la condition que lorque R₈ est un groupe alkyle, il est fixé sur l'atome de silicium de Ar₂.

11. Composé de formule (I) selon la revendication 1, **caractérisé en ce que** :
- A est une liaison directe ;
- Ar, est une hétéroaryle ;
- Ar₂ est un hétérocycloalkyle ;
- R₃ représente un atome d'hydrogène,
- R₄ représente un groupe OH ou -CONH₂,
- R₈ représente un groupe alkyle, alkyl-Si(alkyle)₃; -SO₂-alkyl-Si(alkyle)₃ ; phényle ; alcoxy-imino ; hétérocycloalkyle substitué par un ou plusieurs atomes d'halogène, un ou plusieurs groupes hydroxy ou hydroxy-alkyle ; ou bien R₈ et R₉ ensemble avec l'atome de carbone auquels ils sont liés forment un groupe cycloalkyle éventuellement substitué par un ou plusieurs atomes d'halogène ou un ou plusieurs groupes carboxy ;
- R₉ représente un atome d'hydrogène ou un groupe alkyle ;
à la condition que lorque R₈ est un groupe alkyle, il est fixé sur l'atome de silicium de Ar₂.
à l'état de base ou d'acide ou de sel d'addition à un acide ou à une base.

12. Composé de formule (I) selon l'une quelconque des revendications 11, **caractérisé en ce que** R₈ représente un groupe alkyle, alkyle-Si(alkyle)₃; -SO₂-alkyle-Si(alkyle)₃ ; phényle ; alcoxy-imino ; pyrrolidinyle éventuellement substitué par un ou plusieurs atomes d'halogène, un groupe hydroxy ou hydroxy-alkyle ; thiomorpholinyle ; ou bien R₈ et R₉ ensemble avec l'atome de carbone auquels ils sont liés forment un groupe cycloalkyle éventuellement substitué par un ou plusieurs atomes d'halogène ou un ou plusieurs groupes carboxy ;
- R₉ représente un atome d'hydrogène ou un groupe alkyle ;
à la condition que lorque R₈ est un groupe alkyle, il est fixé sur l'atome de silicium de Ar₂.

13. Composé de formule (I) selon la revendication 1, **caractérisé en ce qu'**il est choisi parmi :
Acide Trans 4-[5-(4-Trimethylsilanylmethyl-piperazin-1-yl)-pyridin-2-yl]-3,4-dihydro-2H-quinoxaline-1-carboxylique (5-hydroxy-adamantan-2-yl)-amide ;
Acide Trans 4-(4-tert-Butoxyimino-3,4,5,6-tetrahydro-2H-[1,3']bipyridinyl-6'-yl)-3,4-dihydro-2H-quinoxaline-1-carboxylique (5-hydroxy-adamantan-2-yl)-amide ;
Acide Trans 4-{5-[4-(2-Trimethylsilanyl-ethanesulfonyl)-piperazin-1-yl]-pyridin-2-yl}-3,4-dihydro-2H-quinoxaline-1-carboxylique (5-hydroxy-adamantan-2-yl)-amide ;
Acide Trans 4-{5-[4-(2,2-Difluoro-cyclopropylmethyl)-piperazin-1-yl]-pyridin-2-yl}-3,4-dihydro-2H-quinoxaline-1-carboxylique (5-carbamoyl-adamantan-2-yl)-amide ;
Acide Trans 4-[4-(1,1-Dioxo-1lambda6-thiomorpholin-4-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans4-[4-((R)-2-Hydroxymethyl-pyrrolidin-1-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans 4-[4-((S)-2-Hydroxymethyl-pyrrolidin-1-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans4-[4-((S)-3-Hydroxy-pyrrolidin-1-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans 4-[4-((R)-3-Hydroxy-pyrrolidin-1-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans 4-[4-(3,3-Difluoro-pyrrolidin-1-yl)-3,4,5,6-tetrahydro-2H-[1,3']bipyridinyl-6'-yl]-3,4-dihydro-2H-quinoxaline-1-carboxylique (5-carbamoyl-adamantan-2-yl)-amide ;
Acide Trans 4-[5-(1,1-Difluoro-6-aza-spiro[2.5]oct-6-yl)-pyridin-2-yl]-3,4-dihydro-2H-quinoxafine-1-carboxylique (5-carbamoyl-adamantan-2-yl)-amide ;
Acide Trans 4-[5-(4-Methyl-4-phenyl-[1,4]azasilinan-1-yl)-pyridin-2-yl]-3,4-dihydro-2H-quinoxaline-1-carboxylique (5-carbamoyl-adamantan-2-yl)-amide ;
Acide Trans 4-[5-(4,4-Dimethyl-[1,4]azasilinan-1-yl)-pyridin-2-yl]-3,4-dihydro-2H-quinoxaline-1-carboxylique (5-carbamoyl-adamantan-2-yl)-amide ;

14. Médicament, **caractérisé en ce qu'**il comprend un composé de formule (I) selon l'une quelconque des revendications 1 à 13, ou un sel d'addition de ce composé à un acide ou à une base pharmaceutiquement acceptable, ou encore un solvat du composé de formule (I).

15. Composition pharmaceutique, **caractérisée en ce qu'**elle comprend un composé de formule (I) selon l'une quelconque des revendications 1 à 13, ou un sel pharmaceutiquement acceptable ou un solvat de ce composé, ainsi qu'au moins un excipient pharmaceutiquement acceptable.

16. Composé de formule (I) selon l'une quelconque des revendications 1 à 13, pour son utilisation dans le traitement et la prévention de l'obésité, des diabètes, des troubles de la microcirculation, de la résistance à l'insuline, du syndrome métabolique, du syndrome de Cushing, de l'hypertension, de l'athérosclérose, de la cognition et de la démence, des glaucomes, de l'ostéoporose, de la lipodystrophie, de l'hypertrophie cardiaque, de l'insuffisance cardiaque, de maladies hépatiques, de certaines maladies infectieuses en augmentant l'efficacité du système immunitaire ou des plaies en favorisant la cicatrisation.

## Patentansprüche

1. Verbindung, entsprechend der Formel (I): 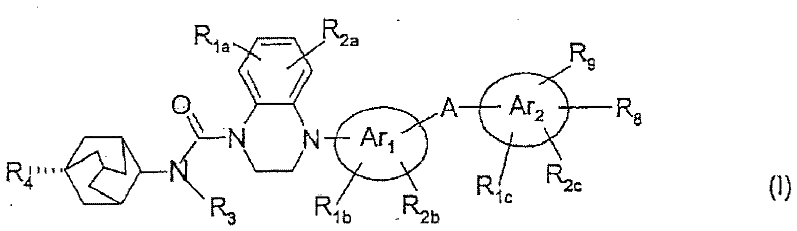 in der:
- A eine Bindung, ein Sauerstoffatom oder eine -KO-CH₂-Gruppe bedeutet,
- Ar₁ eine Phenyl- oder Heteroarylgruppe bedeutet,
- Ar₂ eine Phenylgruppe, eine Heteroarylgruppe oder eine Heterocycloalkylgruppe bedeutet,
- R_{1a,b,c} und R_{2a,b,c}, die gleich oder verschieden sind, jeweils ein Wasserstoff- oder Halogenatom oder eine Alkyl; Cycloalkyl, gegebenenfalls mit einer Alkyl-, Halogenalkyl-, Alkoxyalkyl-, Alkoxyhalogenalkyl- oder COOR₅-Gruppe substituiert; Alkylcycloalkyl, gegebenenfalls mit einem oder mehreren Halogenatomen substituiert; -OR₅ (Hydroxy oder Alkoxy); Hydroxy-Alkyl; Alkoxy-Alkyl; Alkoxy-Alkoxy; Halogenalkyl; O-Halogenalkyl; Oxo; -CO-Alkyl; -CO-Alkyl-NR₆R₇; -CO-Halogenalkyl; -COOR₅; -Alkyl-COOR₅; -O-Alkyl-COOR₅; -SO₂-Alkyl; -SO₂-Cycloalkyl; -SO₂-Alkyl-Cycloalkyl; -SO₂-Alkyl-OR ₅; -SO₂-Alkyl-COOR₅; -SO₂-Alkyl-NR₆R₇; -SO₂-Halogenalkyl; Alkyl-SO₂-Alkyl; -SO₂-NR₆R₇; -SO₂-Alkyl-Alkoxy-Alkoxy; -CONR₆R₇; Alkyl-CONR₆R₇ oder -O-Alkyl-NR₆R₇ bedeuten, oder R₁ₐ, R_{1b}, R_{1c} jeweils an R₂ₐ, R_{2b}, R_{2c} und an das Kohlenstoffatom, das sie trägt, gebunden sind und O-Alkyl-O bedeuten;
- R₃ ein Wasserstoffatom oder eine Alkylgruppe bedeutet,
- R₄ ein Wasserstoff- oder Halogenatom oder eine Cyanogruppe, -OR₅, Hydroxyalkyl, -COOR₅, -NR₆R₇, -CONR₆R₇, -SO₂-Alkyl oder -SO₂-NR₆R₇, -NR₆-COOR₅, -NR₆-COR₅, CO-NR₆-Alkyl-OR₆ bedeutet;
- R₅, R₆ und R₇, die gleich oder verschieden sind, jeweils ein Wasserstoffatom, eine Alkylgruppe oder eine Alkyl-Phenyl-Gruppe bedeuten, und
- R⁸ eine Alkylgruppe, Alkyl-Si(Alkyl)₃; -SO₂-Alkyl-Si(Alkyl)₃; Phenyl-; Alkoxy-Imino-; Alkyl-Cycloalkyl, gegebenenfalls mit einem oder mehreren Halogenatomen substituiert; Heterocycloalkyl mit einem oder mehreren Halogenatomen, einer oder mehreren Hydroxy- oder Hydroxy-alkylgruppen substituiert; oder R₈ und R₉ zusammen mit dem Kohlenstoffatom an das sie gebunden sind, eine Cycloalkylgruppe, gegebenenfalls mit einem oder mehreren Halogenatomen oder einer oder mehreren Carboxygruppen substituiert ist, bilden;
- R₉ ein Wasserstoffatom oder eine Alkylgruppe bedeutet;
unter der Bedingung, dass wenn R₈ eine Alkylgruppe ist, es an das Siliciumatom von Ar₂ gebunden ist,
in Form einer Base oder Säure oder eines Säure- oder Basenadditionssalzes.

2. Verbindung der Formel (I) nach Anspruch 1, **dadurch gekennzeichnet, dass** A eine Bindung bedeutet.

3. Verbindung der Formel (I) nach einem der Ansprüche 1 oder 2, **dadurch gekennzeichnet, dass** Ar₁ eine Heteroarylgruppe bedeutet.

4. Verbindung der Formel (I) nach Anspruch 3, **dadurch gekennzeichnet, dass** Ar₁ eine Pyridinylgruppe bedeutet.

5. Verbindung der Formel (I) nach einem der Ansprüche 1 bis 4, **dadurch gekennzeichnet, dass** Ar₂ eine Heterocycloalkylgruppe bedeutet.

6. Verbindung der Formel (I) nach Anspruch 5, **dadurch gekennzeichnet, dass** Ar₂ die Piperidinyl-, Piperazinyl- oder Azasilinanylegruppe bedeutet.

7. Verbindung der Formel (I) nach einem der Ansprüche 1 bis 6, **dadurch gekennzeichnet, dass** R_{1a,} R_{2a,} R_{1b} und R_{2b} jeweils ein Wasserstoffatom bedeuten.

8. Verbindung der Formel (I) nach einem der Ansprüche 1 bis 7, **dadurch gekennzeichnet, dass** R₃ ein Wasserstoffatom bedeutet.

9. Verbindung der Formel (I) nach einem der Ansprüche 1 bis 8, **dadurch gekennzeichnet, dass** R₄ eine Hydroxy- oder -CONH₂-Gruppe bedeutet.

10. Verbindung der Formel (I) nach einem der Ansprüche 9, **dadurch gekennzeichnet, dass** R₈ eine Alkylgruppe, Alkyl-Si(Alkyl)₃; -SO₂-Alkyl-Si(Alkyl)₃; Phenyl; Alkoxy-imino; Heterocycloalkyl mit einem oder mehreren Halogenatomen, einer oder mehreren Hydroxy- oder Hydroxy-AlkylGruppen substituiert; oder R₈ und R₉ zusammen mit dem Kohlenstoffatom an das sie gebunden sind, eine Cycloalkylgruppe, gegebenenfalls mit einem oder mehreren Halogenatomen oder einer oder mehreren CarboxyGruppen substituiert ist, bilden;
- R₉ ein Wasserstoffatom oder eine Alkylgruppe bedeutet;
unter der Bedingung, dass wenn R₈ eine Alkylgruppe ist, es an das Siliciumatom von Ar₂ gebunden ist.

11. Verbindung der Formel (I) nach Anspruch 1, **dadurch gekennzeichnet, dass**:
- A eine direkte Bindung ist;
- Ar₁ eine Heteroaryl ist;
- Ar₂ eine Heterocycloalkyl ist;
- R₃ ein Wasserstoffatom bedeutet;
- R₄ eine OH- oder -CONH₂-Gruppe bedeutet,
- R₈ eine Alkylgruppe, Alkyl-Si(Alkyl)₃; -SO₂-Alkyl-Si(Alkyl)₃; Phenyl; Alkoxy-Imino; Heterocycloalkyl mit einem oder mehreren Halogenatomen, einer oder mehreren Hydroxy- oder Hydroxy-Alkylgruppen substituiert, bedeutet; oder R₈ und R₉ zusammen mit dem Kohlenstoffatom an das sie gebunden sind, eine Cycloalkylgruppe gegebenenfalls mit einem oder mehreren Halogenatomen oder einer oder mehreren Carboxygruppen substituiert ist, bilden;
- R₉ ein Wasserstoffatom oder eine Alkylgruppe bedeutet;
unter der Bedingung, dass wenn R₈ eine Alkylgruppe ist, es an das Siliciumatom von Ar₂ gebunden ist,
in Form einer Base oder Säure oder eines Säure- oder Basenadditionssalzes.

12. Verbindung der Formel (I) nach einem der Ansprüche 11, **dadurch gekennzeichnet, dass** R₈ eine Alkylgruppe, Alkyl,-Si(Alkyl)₃; -SO₂-Alkyl-Si (Alkyl)₃; Phenyl; Alkoxy-Imino; Pyrrolidinyl gegebenenfalls mit einem oder mehreren Halogenatomen, Hydroxy- oder Hydroxy-Alkylgruppe substituiert; Thiomorpholinyl, bedeutet; oder R₈ und R₉ zusammen mit dem Kohlenstoffatom an das sie gebunden sind, eine Cycloalkylgruppe gegebenenfalls mit einem oder mehreren Halogenatomen oder einer oder mehreren Carboxygruppen substituiert ist, bilden;
- R₉ ein Wasserstoffatom oder eine Alkylgruppe bedeutet;
unter der Bedingung, dass wenn R₈ eine Alkylgruppe ist, es an das Siliciumatom von Ar₂ gebunden ist.

13. Verbindung der Formel (I) nach Anspruch 1, **dadurch gekennzeichnet, dass** sie ausgewählt ist aus:
Trans-4-[5-(4-Trimethylsilanylmethyl-Piperazin-1-yl)-Pyridin-2-yl]-3,4-Dihydro-2H-Chinoxalin-1-Carbonsäure-(5-Hydroxy-Adamantan-2-yl)-Amid;
Trans-4-(4-tert-Butoxyimino-3,4,5,6-Tetrahydro-2H-[1,3']bipyridinyl-6'-yl)-3, 4-Dihydro-2H-Chinoxalin-1-Carbonsäure-(5-Hydroxy-Adamantan-2-yl)-Amid;
Trans-4-{5-[4-(2-Trimethylsilanyl-Ethansulfonyl)-Piperazin-1-yl]-Pyridin-2-yl)-3,4-Dihydro-2H-Chinoxalin-1-Carbonsäure-(5-Hydroxy-Adamantan-2-yl)-Amid;
Trans-4-{5-[4-(2,2-Difluor-Cyclopropylmethyl)-Piperazin-1-yl]-Pyridin-2-yl}-3,4-Dihydro-2H-Chinoxalin-1-Carbonsäure-(5-Carbamoyl-Adamantan-2-yl)-Amid;
Trans-4-[4-(1,1-Dioxo-1 lambda6-Thiomorpholin-4-yl)-3,4,5,6-Tetrahydro-2H-[1,3']bipyridinyl-6'-yl]-3,4-Dihydro-2H-Chinoxalin-1-Carbonsäure-(5-Carbamoyl-Adamantan-2-yl)-Amid;
Trans-4-[4-((R)-2-Hydroxymethyl-Pyrrolidin-1-yl)-3,4,5,6-Tetrahydro-2H-[1,3']bipyridinyl-6'-yl]-3,4-Dihydro-2H-Chinoxalin-1-Carbonsäure-(5-Carbamoyl-Adamantan-2-yl)-Amid;
Trans-4-[4-((S)-2-Hydroxymethyl-Pyrrolidin-1-yl)-3,4,5,6-Tetrahydro-2H-[1,3']bipyridinyl-6'-yl]-3,4-Dihydro-2H-Chinoxalin-1-Carbonsäure-(5-Carbamoyl-Adamantan-2-yl)-Amid;
Trans-4-[4-((S)-3-Hydroxy-pyrrolidin-1-yl)-3,4,5,6-Tetrahydro-2H-[1,3']bipyridinyl-6'-yl]-3,4-Dihydro-2H-Chinoxalin-1-Carbonsäure-(5-Carbamoyl-Adamantan-2-yl)-Amid;
Trans-4-[4-((R)-3-Hydroxy-pyrrolidin-1-yl)-3,4,5,6-Tetrahydro-2H-[1,3']bipyridinyl-6'-yl]-3,4-Dihydro-2H-Chinoxalin-1-Carbonsäure-(5-Carbamoyl-Adamantan-2-yl)-Amid;
Trans-4-[4(3,3-Difluor-Pyrrolidin-1-yl)-3,4,5,6-Tetrahydro-2H-[1,3']bipyridinyl-6'-yl]-3,4-Dihydro-2H-Chinoxalin-1-Carbonsäure-(5-Carbamoyl-Adamantan-2-yl)-Amid;
Trans-4-[5-(1,1-Difluor-6-Aza-Spiro[2.5]oct-6-yl)-Pyridin-2-yl]-3,4-Dihydro-2H-Chinoxalin-1-Carbonsäure(5-Carbamoyl-Adamantan-2-yl)-Amid;
Trans-4-[5-(4-Methyl-4-Phenyl-[1,4]Azasilinan-1-yl)-Pyridin-2-yl]-3,4-Dihydro-2H-Chinoxalin-1-Carbonsäure-(5-Carbamoyl-Adamantan-2-yl)-Amid;
Trans-4-[5-(4,4-Dimethyl-[1,4]-Azasilinan-1-yl)-Pyridin-2-yl]-3,4-Dihydro-2H-Chinoxalin-Carbonsäure-(5-Carbamoyl-Adamantan-2-yl)-Amid.

14. Arzneimittel, **dadurch gekennzeichnet, dass** es eine Verbindung der Formel (I) nach einem der Ansprüche 1 bis 13 oder ein pharmazeutisch annehmbares Säure- oder Basenadditionssalz dieser Verbindung, oder ein Solvat der Verbindung der Formel (I) umfasst.

15. Pharmazeutische Zusammensetzung, **dadurch gekennzeichnet, dass** sie eine Verbindung der Formel (I) nach einem der Ansprüche 1 bis 13 oder ein pharmazeutisch annehmbares Salz oder ein Solvat dieser Verbindung sowie mindestens einen pharmazeutisch annehmbaren Hilfsstoff umfasst

16. Verbindung der Formel (I) nach einem der Ansprüche 1 bis 13 zur Verwendung bei der Behandlung und Vorbeugung von Obesitas, Diabetes, Mikrozirkulationsstörungen, Insulinresistenz, metabolischem Syndrom, Cushing-Syndrom, Bluthochdruck, Arteriosklerose, Kognition und Demenz, Glaukomen, Osteoporose, Lipodystrophie, Herzhypertrophie, Herzinsuffizienz, Lebererkrankungen, bestimmten Infektionserkrankungen durch die Erhöhung der Effizienz des Immunsystems oder die Förderung der Wundheilung.

## Claims

1. A compound corresponding to formula (I): 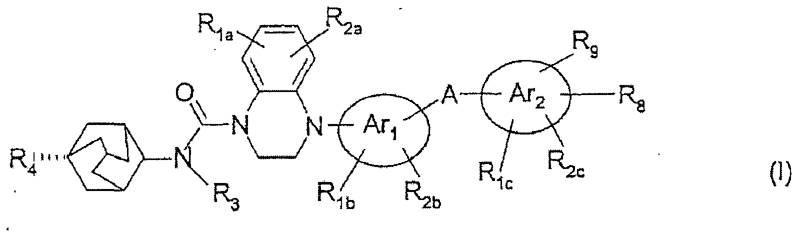 in which:
- A represents a bond, an oxygen atom or an O-CH₂-group,
- Ar₁ represents a phenyl or heteroaryl group,
- Ar₂ is a phenyl group, a heteroaryl group or a heterocycloalkyl group,
- R_{1a,b,c} and R_{2a,b,c} , which may be identical or different, each represent hydrogen or halogen atom or an alkyl group; cycloalkyl optionally substituted with an alkyl, haloalkyl, alkoxy-alkyl, alkoxy-haloalkyl or - COOR₅ group; -alkyl-cycloalkyl optionally substituted with one or more halogen atoms; -OR₅ (hydroxy or alkoxy); hydroxy-alkyl; alkoxy-alkyl; alkoxy-alkoxy; haloalkyl; -O-haloalkyl; oxo; -CO-alkyl; -CO-alkyl-NR₆R₇; -CO-haloalkyl; -COOR₅; alkyl-COOR₅; -O-alkyl-COOR₅; -SO₂-alkyl; -SO₂-cycloalkyl; -SO₂-alkyl-cycloalkyl; -SO₂-alkyl-OR₅; -SO₂-alkyl-COOR₅; -SO₂-alkyl-NR₆R₇; -SO₂-haloalkyl; alkyl-SO₂-alkyl; -SO₂-NR₆R₇; -SO₂-alkyl-alkoxy-alkoxy; -CONR₆R₇; alkyl-CONR₆R, or -O-alkyl-NR₆R₇, or R₁ₐ, R_{1b}, R_{1c} are bound respectively to R₂ₐ, R_{2b}, R_{2c} and to the carbon atom which bears them and represent -O-alkyl-O;
- R₃ represents a hydrogen atom or an alkyl group,
- R₄ represents a hydrogen or halogen atom or a cyano, -OR₅, hydroxylalkyl, -COOR₅, -NR₆R₇, -CONR₆R₇, -SO₂-alkyl or -SO₂-NR₆R₇, -NR₆-COOR₅, -NR₆-COR₅, -CO-NR₆-alkyl-OR₅ group;
- R₅, R₆ and R₇, which may be identical or different, each represent a hydrogen atom, an alkyl group or a alkyl-phenyl group, and
- R₈ represents an alkyl group, alkyl-Si(alkyl)₃; -SO₂-alkyl-Si(alkyl)₃; phenyl; alkoxy-imino; alkyl-cycloalkyl optionally substituted with one or more halogen atoms; heterocycloalkyl substituted with one or more halogen atoms, one or more hydroxyl or hydroxy-alkyl groups; or else R₈ and R₉, together with the carbon atom to which they are bound, form a cycloalkyl group optionally substituted with one or more halogen atoms or one or more carboxyl groups;
- R₉ represents a hydrogen atom or an alkyl group; provided that when R₈ is an alkyl group, it is attached to the silicium atom of Ar₂,
in the form of base or acid or salt of addition to an acid or a base.

2. The compound of formula (I) according to claim 1, **characterized in that** A represents a bond.

3. The compound of formula (I) according to one of claims 1 or 2, **characterized in that** Ar₁ represents a heteroaryl group.

4. The compound of formula (I) according to claim 3, **characterized in that** Ar₁ represents the pyridinyl group.

5. The compound of formula (I) according to any one of claims 1 to 4, **characterized in that** Ar₂ represents a heterocycloalkyl group.

6. The compound of formula (I) according to claim 5, **characterized in that** Ar₂ represents the piperidinyl, piperazinyl or azasilinanyl group.

7. The compound of formula (I) according to any one of claims 1 to 6, **characterized in that** R₁ₐ, R₂ₐ, R_{1b} and R_{2b} each represent a hydrogen atom.

8. The compound of formula (I) according to any one of claims 1 to 7, **characterized in that** R₃ represents a hydrogen atom.

9. The compound of formula (I) according to any one of claims 1 to 8, **characterized in that** R₄ represents a hydroxyl or -CONH₂ group.

10. The compound of formula (I) according to any one of claims 9, **characterized in that** R₈ is an alkyl group, alkyl-Si(alkyl)₃; -SO₂-alkyl-Si (alkyl)₃; phenyl; alkoxy-imino; heterocycloalkyl substituted with one or more halogen atoms, one or more hydroxyl or hydroxy-alkyl groups; or else R₈ and R₉, together with the carbon atom to which they are bound, form a cycloalkyl group optionally substituted with one or more halogen atoms or one or more carboxyl groups;
- R₉ represents a hydrogen atom or an alkyl group; provided that when R₈ is an alkyl group, it is attached to the silicon atom of Ar₂.

11. The compound of formula (I) according to claim 1, **characterized in that**:
- A is a direct bond;
- Ar₁ is a heteroaryl;
- Ar₂ is a heterocycloalkyl;
- R₃ represents a hydrogen atom,
- R₄ represents an OH or -CONH₂ group,
- R₈ represents an alkyl group, alkyl-Si(alkyl)₃; -SO₂-alkyl-Si(alkyl)₃; phenyl; alkoxy-imino; heterocycloalkyl substituted with one or more halogen atoms, one or more hydroxyl or hydroxy-alkyl groups; or else R₈ and R₉, together with the carbon atom to which they are bound, form a cycloalkyl group optionally substituted with one or more halogen atoms or one or more carboxyl groups;
- R₉ represents a hydrogen atom or an alkyl group;
provided that when R₈ is an alkyl group, it is attached to the silicium atom of Ar₂,
in the form of base or acid or of salt of addition to an acid or a base.

12. The compound of formula (I) according to any one of claims 11, **characterized in that** R₈ represents an alkyl group, alkyl-Si(alkyl)₃; -SO₂-alkyl-Si(alkyl)₃; phenyl; alkoxy-imino; pyrrolidinyl optionally substituted with one or more halogen atoms, a hydroxyl or hydroxy-alkyl group; thiomorpholinyl group; or R₈ and R₉, together with the carbon atom to which they are bound, form a cycloalkyl group optionally substituted with one or more halogen atoms or one or more carboxyl groups;
- R₉ represents a hydrogen atom or an alkyl group;
provided that when R₈ is an alkyl group, it is attached to the silicon atom of Ar₂.

13. The compound of formula (I) according to claim 1, **characterized in that** it is chosen from:
Trans-4-[5-(4-trimethylsilanylmethyl-piperazin-1-yl)-pyridin-2-yl]-3,4-dihydro-2H-quinoxaline-1-carboxylic acid (5-hydroxy-adamantan-2-yl)-amide;
Trans-4-(4-tert-butoxyimino-3,4,5,6-tetrahydro-2H-[1,3']bipyridinyl-6'-yl)-3,4-dihydro-2H-quinoxaline-1-carboxylic acid (5-hydroxy-adamantan-2-yl) amide;
Trans-4-{5-[4-(2-trimethylsilanyl-ethansulfonyl)-piperazin-1-yl]-pyridin-2-yl}-3,4-dihydro-2H-quinoxaline-1 -carboxylic acid (5-hydroxy-adamantan-2-yl) amide;
Trans-4-{5-[4-(2,2-difluor-cyclopropylmethyl)-piperazin-1-yl]-pyridin-2-yl}-3,4-dihydro-2H-quinoxaline-1-carboxylic acid (5-carbamoyl-adamantan-2-yl) amide;
Trans-4-[4-(1,1-dioxo-1lambda6-thiomorpholin-4-yl)-3,4,5,6-tetrahydro-2H-[1,3']bipyridinyl-6'-yl]-3,4-dihydro-2H-quinoxaline-1-carboxylic acid (5-carbamoyl-adamantan-2-yl) amide;
Trans-4-[4-((R)-2-hydroxymethyl-pyrrolidin-1-yl)-3,4,5,6-tetrahydro-2H-[1,3']bipyridinyl-6'-yl]-3,4-dihydro-2H-quinoxaline-1-carboxylic acid (5-carbamoyl-adamantan-2-yl) amide;
Trans-4-[4-((S)-2-hydroxymethyl-pyrrolidin-1-yl)-3,4,5,6-tetrahydro-2H-[1,3']bipyridinyl-6'-yl]-3,4-dihydro-2H-quinoxaline-1-carboxyfic acid (5-carbamoyl-adamantan-2-yl) amide;
Trans-4-[4-((S)-3-hydroxy-pyrrolidin-1-yl)-3,4,5,6-tetrahydro-2H-[1,3']bipyridinyl-6'-yl]-3,4-dihydro-2H-quinoxaline-1-carboxylic acid (5-carbamoyl-adamantan-2-yl) amide;
Trans-4-[4-((R)-3-hydroxy-pyrrolidin-1-yl)-3,4,5,6-tetrahydro-2H-[1,3']bipyridinyl-6'-yl]-3,4-dihydro-2H-quinoxaline-1-carboxylic acid (5-carbamoyl-adamantan-2-yl) amide;
Trans-4-[4(3,3-difluor-pyrrolidin-1-yl)-3,4,5,6-tetrahydro-2H-[1,3']bipyridinyl-6'-yl]-3,4-dihydro-2H-quinoxaline-1-carboxylic acid (5-carbamoyl-adamantan-2-yl) amide;
Trans-4-[5-(1,1-difluor-6-aza-spiro[2.5]oct-6-yl)-pyridin-2-yl]-3,4-dihydro-2H-quinoxaline-1-carboxylic acid (5-carbamoyl-adamantan-2-yl) amide;
Trans-4-[5-(4-methyl-4-phenyl-[1,4]azasilinan-1-yl)-pyridin-2-yl]-3,4-dihydro-2H-quinoxaline-1-carboxylic acid (5-carbamoyl-adamantan-2-yl) amide;
Trans-4-[5-(4,4-dimethyl-[1,4]-azasilinan-1-yl)-pyridin-2-yl]-3,4-dihydro-2H-quinoxaline-carboxylic acid (5-carbamoyl-adamantan-2-yl) amide.

14. Medicament, **characterized in that** it comprises the compound of formula (I) according to any one of claims 1 to 13 or an addition salt thereof to a pharmaceutically acceptable acid or base, or alternatively a solvate of the compound of formula (I).

15. Pharmaceutical composition, **characterized in that** it comprises the compound of formula (I) according to any one of claims 1 to 13, or a pharmaceutically acceptable salt or a solvate of this compound, and at least one pharmaceutically acceptable excipient.

16. The compound of formula (I) according to any one of claims 1 to 13 for use in the treatment and prevention of obesity, diabetes, disorders of the microcirculation, insulin resistance, metabolic syndrome, Cushing's syndrome, hypertension, atherosclerosis, cognition and dementia, glaucoma, osteoporosis, lipodystrophy, cardiac hypertrophy, heart failure, liver diseases, certain infectious diseases by increasing the effectiveness of the immune system or wounds by promoting healing.
